# EUROPEAN PATENT APPLICATION

(11) **EP 3 342 864 A1**
(43) Date of publication of application: **04.07.2018**
(21) Application number: 16839378.3
(22) Date of filing: 26.08.2016
(51) Int. Cl.: C12N 15/09, A61K 35/74, A61K 35/744, A61K 35/745, A61K 45/00, A61P 37/02, C12Q 1/06, C12Q 1/68

(54) **AUTOIMMUNE DISEASE DIAGNOSIS METHOD, AUTOIMMUNE DISEASE DIAGNOSIS BIOMARKER, AND AUTOIMMUNE DISEASE PREVENTING OR TREATING AGENT**

(30) Priority: 27.08.2015 JP 2015167839
(71) Applicant: National Center of Neurology and Psychiatry, Kodaira-shi Tokyo 187-8551 (JP); The University Of Tokyo, Tokyo 113-8654 (JP); School Corporation, Azabu Veterinary Medicine Educational Institution, Sagamihara-shi, Kanagawa 252-5201 (JP); Juntendo Educational Foundation, Tokyo 113-8421 (JP)
(72) Inventor: YAMAMURA, Takashi, Kodaira-shi Tokyo 187-8551 (JP); HATTORI, Masahira, Tokyo 113-8654 (JP); MORITA, Hidetoshi, Sagamihara-shi Kanagawa 252-5201 (JP); MIYAKE, Sachiko, Tokyo 113-8421 (JP)
(74) Representative: Hoffmann Eitle
(86) International application number: PCT/JP2016/075070
(87) International publication number: WO 2017/034031

(57) **Abstract**

Provided is a diagnosis method for an autoimmune disease, including a step of measuring the relative abundances of bacteria included in a fecal sample collected from a test subject; and a step of performing the following (1) or (2): (1) in a case in which relative abundance of a bacterium whose nucleotide sequence of 16S ribosomal RNA gene has an identity of 99% or higher with nucleotide sequence set forth in SEQ ID NO:3 or SEQ ID NO:4, is large compared to the relative abundance in healthy subject, determining that the test subject has contracted, or has a high risk of contracting, the autoimmune disease; and (2) in a case in which relative abundance of a bacterium whose nucleotide sequence of 16S ribosomal RNA gene has an identity of 99% or higher with any one of nucleotide sequences set forth in SEQ ID NO:5 to SEQ ID NO:23, is small compared to the relative abundance in healthy subject, determining that the test subject has contracted, or has a high risk of contracting, the autoimmune disease.

## Description

### Technical Field

The present invention relates to a diagnosis method for an autoimmune disease, a biomarker for autoimmune disease diagnosis, and a preventing or treating agent for an autoimmune disease.

### Background Art

Multiple sclerosis (MS) is one of autoimmune diseases, and this is a disease that causes nerve conduction disorders, in which multiple inflammation targeted at myelin sheath and nerve axons is brought about and leads to extensive demyelination.

In recent years, it has become obvious that the intestinal bacterial flora is an important factor affecting the cellular and humoral immunity of the intestinal immune system (Non-Patent Literature 1). Furthermore, it has been reported that those bacteria belonging to human feces-derived Clostridium cluster XTVa and cluster IV, and *Bacteroides fragilis* induce Foxp3⁺ regulatory T-cells and suppress inflammatory conditions such as colitis and experimental autoimmune encephalomyelitis (EAE) (Non-Patent Literatures 2 to 4).

### Citation List

### Non Patent Literature

[Non-Patent Literature 1] Cell, 2014, Vol. 157, pp. 121-141
[Non-Patent Literature 2] Science, 2011, Vol. 331, pp. 337-341
[Non-Patent Literature 3] J. Immunol., 2010, Vol. 185, pp. 4101-4108
[Non-Patent Literature 4] Nature, 2013, Vol. 500, pp. 232-236

### Summary of Invention

### Problems to be Solved by the Invention

An object of the present invention is to clarify the correlation between the intestinal bacterial flora and autoimmune diseases such as MS, and to provide a diagnosis method for an autoimmune disease based on this correlation. Another object of the present invention is to provide a biomarker for autoimmune disease diagnosis and a treating agent for an autoimmune disease.

### Means for Solving the Problems

The inventors of the present invention found that there is a statistically significant difference between the compositions of the intestinal bacterial florae of MS patients and healthy controls. Furthermore, the inventors found bacterial species whose relative abundances in the intestinal bacterial florae are statistically significantly different between MS patients and healthy controls. The invention is based on these findings.

That is, the invention relates to, for example, inventions according to the following items [1] to [8].
[1] A diagnosis method for an autoimmune disease, including:
   a step of measuring relative abundances of bacteria included in a fecal sample collected from a test subject; and
   a step of performing the following (1) or (2):
      (1) in a case in which relative abundance of a bacterium whose nucleotide sequence of 16S ribosomal RNA gene has an identity of 99% or higher with nucleotide sequence set forth in SEQ ID NO:3 or SEQ ID NO: 4, is large compared to the relative abundance in healthy subject, determining that the test subject has contracted, or has a high risk of contracting, the autoimmune disease; and
      (2) in a case in which relative abundance of a bacterium whose nucleotide sequence of 16S ribosomal RNA gene has an identity of 99% or higher with any one of nucleotide sequences set forth in SEQ ID NO:5 to SEQ ID NO:23, is small compared to the relative abundance in healthy subject, determining that the test subject has contracted, or has a high risk of contracting, the autoimmune disease.
[2] A diagnosis method for an autoimmune disease, including:
   a step of measuring relative abundances of bacteria included in a fecal sample collected from a test subject before treatment and after treatment; and
   a step of performing the following (3) or (4):
      (3) in a case in which relative abundances before and after treatment of a bacterium whose nucleotide sequence of 16S ribosomal RNA gene has an identity of 99% or higher with nucleotide sequence set forth in SEQ ID NO:3 or SEQ ID NO:4 are compared, and the relative abundance after treatment is small compared to the relative abundance before treatment, determining that the disease state of the autoimmune disease of the test subject has been ameliorated by the treatment; and
      (4) in a case in which relative abundances before and after treatment of a bacterium whose nucleotide sequence of 16S ribosomal RNA gene has an identity of 99% or higher with any one of nucleotide sequences set forth in SEQ ID NO:5 to SEQ ID NO:23 are compared, and the relative abundance after treatment is large compared to the relative abundance before treatment, determining that the disease state of the autoimmune disease of the test subject has been ameliorated by the treatment.
[3] The diagnosis method according to [1] or [2], in which the measurement of the relative abundances of the bacteria includes comprehensive decoding of the nucleotide sequence of 16S ribosomal RNA gene of the bacterium included in the fecal sample.
[4] The diagnosis method according to any one of [1] to [3], in which the autoimmune disease is multiple sclerosis.
[5] The diagnosis method according to [4], in which the multiple sclerosis is relapsing-remitting multiple sclerosis.
[6] A biomarker for autoimmune disease diagnosis, including an intestinal bacterium whose nucleotide sequence of 16S ribosomal RNA gene has an identity of 99% or higher with any one of nucleotide sequences set forth in SEQ ID NO:3 to SEQ ID NO:23.
[7] Use of an intestinal bacterium whose nucleotide sequence of 16S ribosomal RNA gene having an identity of 99% or higher with any one of nucleotide sequences set forth in SEQ ID NO:3 to SEQ ID NO:23, as a biomarker for autoimmune disease diagnosis.
[8] A preventing or treating agent for an autoimmune disease, including, as an active ingredient, at least one selected from the group consisting of a bacterium whose nucleotide sequence of 16S ribosomal RNA gene has an identity of 99% or higher with any one of the nucleotide sequences set forth in SEQ ID NO:5 to SEQ ID NO:23; and a physiologically active substance derived from the bacterium.

The invention also relates to the following items [2-1] to [2-5].
[2-1] A computer-readable non-transitory recording medium storing a program that causes a computer to execute: a step of obtaining nucleotide sequence data by comprehensively decoding the nucleotide sequences of 16S ribosomal RNA gene of bacteria included in a fecal sample collected from a test subject; a step of calculating the frequency of a nucleotide sequence having an identity of 99% or higher with the nucleotide sequence set forth in any one of SEQ ID NO:3 to SEQ ID NO:23 from the nucleotide sequence data thus obtained, and calculating the relative abundance of the nucleotide sequence; a step of comparing the relative abundance thus calculated with a reference value that has been inputted in advance, and determining the disease state of an autoimmune disease; and a step of outputting the determination result thus obtained.
[2-2] A computer-readable non-transitory recording medium storing a program that causes a computer to execute: a step of obtaining nucleotide sequence data by comprehensively decoding the nucleotide sequences of 16S ribosomal RNA gene of bacteria included in a fecal sample collected from a test subject; a step of calculating the frequency of a nucleotide sequence having an identity of 99% or higher with the nucleotide sequence set forth in any one of SEQ ID NO:3 to SEQ ID NO:23 from the nucleotide sequence data thus obtained, and calculating the relative abundance of the nucleotide sequence; a step of comparing the relative abundance thus calculated with the relative abundance in healthy subject, which has been inputted in advance; a step of determining that the test subject has contracted, or has a high risk of contracting, an autoimmune disease based on the comparison results; and a step of outputting the determination result thus obtained, wherein in the determining step, in a case in which the above-mentioned relative abundance is the relative abundance of a nucleotide sequence having an identity of 99% or higher with the nucleotide sequence set forth in SEQ ID NO:3 or SEQ ID NO:4, and the relative abundance thus calculated is large compared to the relative abundance in the healthy subject, or in a case in which the above-mentioned relative abundance is the relative abundance of a nucleotide sequence having an identity of 99% or higher with the nucleotide sequences set forth in SEQ ID NO:5 to SEQ ID NO:23, and the relative abundance thus calculated is small compared to the relative abundance in the healthy subject, it is determined that the test subject has contracted, or has a high risk of contracting, the autoimmune disease.
[2-3] A computer-readable non-transitory recording medium storing a program that causes a computer to execute: a step of obtaining nucleotide sequence data by comprehensively decoding the nucleotide sequences of 16S ribosomal RNA gene of bacteria included in a fecal sample collected from a test subject after treatment; a step of calculating the frequency of a nucleotide sequence having an identity of 99% or higher with the nucleotide sequence set forth in any one of SEQ ID NO:3 to SEQ ID NO:23 from the nucleotide sequence data thus obtained, and calculating the relative abundance of the nucleotide sequence; a step of comparing the relative abundance thus calculated with the relative abundance in the test subject before treatment, which has been inputted in advance; a step of determining whether the disease state of an autoimmune disease of the test subject has been ameliorated by the treatment, based on the comparison results; and a step of outputting the determination result thus obtained, wherein in the determining step, in a case in which the above-mentioned relative abundance is the relative abundance of a nucleotide sequence having an identity of 99% or higher with the nucleotide sequence set forth in SEQ ID NO:3 or SEQ ID NO:4, and the relative abundance thus calculated is large compared to the relative abundance in the test subject before treatment, or in a case in which the above-mentioned relative abundance is the relative abundance of a nucleotide sequence having an identity of 99% or higher with the nucleotide sequences set forth in SEQ ID NO:5 to SEQ ID NO:23, and the relative abundance thus calculated is small compared to the relative abundance in the test subject before treatment, it is determined that the disease state of the autoimmune disease of the test subject has been ameliorated by the treatment.
[2-4] A diagnosis system for an autoimmune disease, including: an input means for obtaining nucleotide sequence data by comprehensively decoding the nucleotide sequences of 16S ribosomal RNA gene of bacteria included in a fecal sample collected from a test subject; a calculation means for determining, based on the nucleotide sequence data thus obtained, whether the test subject has contracted, or has a high risk of contracting, the autoimmune disease; and an output means for outputting the determination result obtained by the calculation means.
[2-5] A diagnosis system for an autoimmune disease, including: an input means for obtaining nucleotide sequence data by comprehensively decoding the nucleotide sequences of 16S ribosomal RNA gene of bacteria included in a fecal sample collected from a test subject after treatment; a calculation means for determining, based on the nucleotide sequence data thus obtained, whether the disease state of the autoimmune disease of the test subject has been ameliorated by treatment; and an output means for outputting the determination result obtained by the calculation means.

### Effects of the Invention

According to the invention, a diagnosis method for an autoimmune disease based on the intestinal bacterial flora can be provided. Furthermore, according to the invention, a biomarker for autoimmune disease diagnosis, and a treating agent for an autoimmune disease can be provided.

### Brief Description of Drawings

Fig. 1 is a set of graphs showing the results of analyzing the intestinal bacterial florae of MS20 group and HC40 group. Fig. 1(a) shows the average values of the number of OTU's and clusters of MS20 group and HC40 group. Fig. 1(b) shows the Chao1 estimates of the number of OTU's and clusters of MS20 group and HC40 group. Fig. 1(c) shows the Shannon values of MS20 group and HC40 group.
Fig. 2 is a set of graphs showing the results of an unweighted UniFrac analysis of the intestinal bacterial florae of MS20 group and HC40 group. Fig. 2(a) shows the results of a principal coordinates analysis (PCoA). Fig. 2(b) shows the results of a UniFrac distance analysis.
Fig. 3 is a set of graphs showing the results of a weighted UniFrac analysis of the intestinal bacterial florae of MS20 group and HC40 group. Fig. 3(a) shows the results of a principal coordinates analysis (PCoA). Fig. 1(b) shows the results of a UniFrac distance analysis.
Fig. 4 is a graph showing the results of analyzing the bacterial species composition in the intestinal bacterial florae of MS20 group and HC40 group at the phylum level.
Fig. 5 is a graph showing the results of analyzing the bacterial species composition in the intestinal bacterial florae of MS20 group and HC40 group at the genus level.
Fig. 6 is a diagram showing the workflow of a mapping analysis of 16S reads.
Fig. 7 is a graph showing the differences in the relative abundances of bacteria (Log₁₀(average number of reads of MS20 group /average number of reads of HC40 group)) between MS20 group and HC40 group.
Fig. 8 is a table showing the results of analyzing the degrees of similarity of the nucleotide sequences of VI-V2 region of 16S ribosomal RNA (rRNA) gene.
Fig. 9 is a table showing the results of analyzing the degrees of similarity of the nucleotide sequences of the V1-V2 region of 16S rRNA gene.
Fig. 10 is a diagram showing the results of a phylogenetic analysis of the bacterial species of Clostridia.
Fig. 11 is a table showing the results of analyzing the degrees of similarity of the nucleotide sequences of the V1-V2 region of 16S rRNA gene.
Fig. 12 is a graph showing the differences in the relative abundances of bacteria (Log₁₀(average number of reads of MS20 group /average number of reads of long-term HC18 group)) between MS20 group and long-term HC18 group.

### Embodiments for Carrying Out the Invention

Hereinafter, embodiments for carrying out the present invention will be described in detail. However, the invention is not intended to be limited to the following embodiments.

The diagnosis method for an autoimmune disease, the biomarker for autoimmune disease diagnosis, the diagnosis program and the diagnosis system for an autoimmune disease, and the treating agent for an autoimmune disease according to the present embodiments are based on a novel finding that in a patient who has contracted multiple sclerosis, which is one of autoimmune diseases, the composition of the intestinal bacterial flora significantly changes compared to a healthy control.

An autoimmune disease is a disease that develops as one's own immune system reacts with one's own healthy cells and tissues. Examples of the autoimmune disease include diseases such as multiple sclerosis, rheumatic arthritis, psoriasis, Crohn's disease, leukoderma vulgaris, Behcet's disease, collagenosis, Type I diabetes mellitus, uveitis, Sjoegren syndrome, autoimmune myocarditis, autoimmune liver diseases, autoimmune gastritis, pemphigus, Guillain-Barre syndrome, chronic inflammatory demyelinating polyneuropathy, and HTLV-1-associated myelopathy.

Multiple sclerosis includes relapsing-remitting MS (RR-MS), in which acute aggravation and remission are repeated, and progressive MS. Progressive MS is known to include primary progressive MS (PP-MS); secondary progressive MS (SP-MS), in which the disease state of RR-MS is continued for a certain time period and then the disease state is switched over to a progressive disease state; and progressive relapsing MS (PR-MS) in which the disease progresses while relapsing is repeated.

The autoimmune disease to which the invention is directed is preferably multiple sclerosis, and more preferably relapsing-remitting multiple sclerosis.

### [Diagnosis method for autoimmune disease]

Since the diagnosis method for an autoimmune disease according to the present embodiments is intended to make a decision based on the composition of the intestinal bacterial flora of a test subject, the diagnosis method can be used for, for example, determining the presence or absence of contraction or a risk of contraction of the autoimmune disease (first embodiment), and determining a therapeutic effect for the autoimmune disease (second embodiment).

The diagnosis method according to the first embodiment includes a step of measuring the relative abundances of bacteria included in a fecal sample collected from a test subject; and a step of performing the following (1) or (2):
(1) in a case in which the relative abundance of a bacterium whose nucleotide sequence of 16S ribosomal RNA gene has an identity of 99% or higher with the nucleotide sequence set forth in SEQ ID NO:3 or SEQ ID NO:4, is large compared to the relative abundance in healthy subject, determining that the test subject has contracted, or has a high risk of contracting, the autoimmune disease;
(2) in a case in which the relative abundance of a bacterium whose nucleotide sequence of 16S ribosomal RNA gene has an identity of 99% or higher with any one of the nucleotide sequences set forth in SEQ ID NO:5 to SEQ ID NO:23, is small compared to the relative abundance in healthy subject, determining that the test subject has contracted, or has a high risk of contracting, the autoimmune disease.

The diagnosis method according to the second embodiment includes a step of measuring the relative abundances of bacteria included in a fecal sample collected from a test subject before treatment and after treatment; and a step of performing the following (3) or (4):
(3) in a case in which the relative abundances before and after treatment of a bacterium whose nucleotide sequence of 16S ribosomal RNA gene has an identity of 99% or higher with the nucleotide sequence set forth in SEQ ID NO:3 or SEQ ID NO:4 are compared, and the relative abundance after treatment is small compared to that before treatment, determining that the disease state of the autoimmune disease of the test subject has been ameliorated by the treatment; and
(4) in a case in which the relative abundances before and after treatment of a bacterium whose nucleotide sequence of 16S ribosomal RNA gene has an identity of 99% or higher with any one of the nucleotide sequence set forth in SEQ ID NO:5 to SEQ ID NO:23 are compared, and the relative abundance after treatment is large compared to that before treatment, determining that the disease state of the autoimmune disease of the test subject has been ameliorated by the treatment.

The relative abundance of a bacterium means the proportion occupied by the (particular) bacterium in the whole bacterial flora. The relative abundance of a bacterium can be determined from, for example, the total number of bacterial cells constituting the bacterial flora and the number of the particular bacterial cells included in the bacterial flora. More specifically, for example, genes having a nucleotide sequence that is common in the bacteria included in the bacterial flora and nucleotide sequences characteristic to each bacterial species (for example, 16S rRNA gene) are comprehensively decoded, and the relative abundance of a particular bacterium can be determined by designating the total number of decoded genes and the total number of genes belonging to particular bacterial species as the total number of bacterial cells constituting the bacterial flora and the number of particular bacterial cells, respectively.

As will be described in detail below in the Examples, as a bacterium whose relative abundance significantly increases in an MS patient compared to a healthy control, a bacterium whose nucleotide sequence of the V1 region-V2 region of 16S rRNA gene is the nucleotide sequence set forth in SEQ ID NO:3 or SEQ ID NO:4 was identified. Similarly, as a bacterium whose relative abundance significantly decreases in an MS patient compared to a healthy control, a bacterium whose nucleotide sequence of the V1 region-V2 region of 16S rRNA gene is the nucleotide sequence set forth in any one of SEQ ID NO:5 to SEQ ID NO:23 was identified.

Therefore, by taking the relative abundance of a bacterium whose nucleotide sequence of 16S ribosomal RNA gene has an identity of 99% or higher with the nucleotide sequence set forth in any one of SEQ ID NO:3 to SEQ ID NO:23 as an index, it is made possible to determine the presence or absence of contraction or a risk of contraction of an autoimmune disease, and to determine a therapeutic effect of an autoimmune disease. From the viewpoint of further increasing the accuracy of determination, the identity is preferably 99.5% or higher, more preferably 99.7% or higher, even more preferably 99.9% or higher, and still more preferably 100%.

The term "identity" as used herein means the proportion of coinciding nucleotides when alignment of two nucleotide sequences (for example, alignment using the BLAST algorithm) is performed.

The 16S rRNA gene of a eubacterium includes regions where the degree of preservation of the nucleotide sequence is high in many species (preservation regions), as well as regions of a nucleotide sequence intrinsic to a particular bacterial species and allied species thereof (variable regions). 16S rRNA gene is known to have nine variable regions called V1 to V9. Bacterial species can be specified by identifying the nucleotide sequences of the variable regions. The nucleotide sequence of the V1 region-V2 region of 16S rRNA gene is the nucleotide sequence of V1 variable region and V2 variable region.

The sample derived from a test subject, which is used for the diagnosis method for an autoimmune disease according to the present embodiment, may be any sample with which the composition of the intestinal bacterial flora of the test subject can be analyzed, and a fecal sample of the test subject can be used. The fecal sample may be feces excreted through the anus of the test subject, or may be feces before excretion collected from the intestines (particularly, large intestine) of the test subject.

In the diagnosis method for an autoimmune disease according to the present embodiment, (1) in a case in which the relative abundance of a bacterium whose nucleotide sequence of 16S ribosomal RNA gene has an identity of 99% or higher with the nucleotide sequence set forth in SEQ ID NO:3 or SEQ ID NO:4, is large compared to the relative abundance in healthy subject, it can be determined that the test subject has contracted, or has a high risk of contracting, the autoimmune disease. Similarly, (2) in a case in which the relative abundance of a bacterium whose nucleotide sequence of 16S ribosomal RNA gene has an identity of 99% or higher with any one of the nucleotide sequences set forth in SEQ ID NO:5 to SEQ ID NO:23, is small compared to the relative abundance in healthy subject, it can be determined that the test subject has contracted, or has a high risk of contracting, the autoimmune disease.

In regard to the determination on the presence or absence of contraction or the risk of contraction of an autoimmune disease, determination may be made for at least one kind of bacterium whose nucleotide sequence of 16S ribosomal RNA gene has an identity of 99% or higher with the nucleotide sequence set forth in any one of SEQ ID NO:3 to SEQ ID NO:23. From the viewpoint of further increasing the accuracy of determination, determination may be made for two or more of the above-described bacterial species, or determination may be made for all of the above-described bacteria.

The relative abundance of the above-described bacterium in healthy subject may be measured in advance. The relative abundance of the bacterium in healthy subject may be an average value of a plurality of healthy subjects. Furthermore, the presence or absence of a significant difference may be analyzed from a plurality of data of the relative abundance of the bacterium in healthy subject and the data of the relative abundance in the test subject, by means of a statistical analysis (for example, Welch's t-test).

In the diagnosis method for an autoimmune disease according to the present embodiment, (3) in a case in which the relative abundances before and after treatment of a bacterium whose nucleotide sequence of 16S ribosomal RNA gene has an identity of 99% or higher with the nucleotide sequence set forth in SEQ ID NO:3 or SEQ ID NO:4, are compared, and the relative abundance after treatment is small compared to that before treatment, it can be determined that the disease state of the autoimmune disease of the test subject has been ameliorated by the treatment. Similarly, (4) in a case in which the relative abundances before and after treatment of a bacterium whose nucleotide sequence of 16S ribosomal RNA gene has an identity of 99% or higher with any one of the nucleotide sequences set forth in SEQ ID NO:5 to SEQ ID NO:23, are compared, and the relative abundance after treatment is large compared to that before treatment, it can be determined that the disease state of the autoimmune disease of the test subject has been ameliorated by treatment.

In regard to the determination on the amelioration of the disease state of an autoimmune disease, determination may be made for at least one kind of bacterium whose nucleotide sequence of 16S ribosomal RNA gene has an identity of 99% or higher with the nucleotide sequence set forth in any one of SEQ ID NO:3 to SEQ ID NO:23. From the viewpoint of further increasing the accuracy of determination, determination may be carried out for two or more of the above-described bacterial species, or determination may be made for all of the above-described bacteria.

The relative abundance of the bacterium in a test subject before treatment may be measured in advance, or may be measured approximately simultaneously with the relative abundance of the bacterium in the test subject after treatment. The terms "before treatment" and "after treatment" as used herein are concepts including, for example, time points before and after a treatment (for example, third administration) applied in the middle of a period during which continuous treatment (for example, regular drug administration) is carried out.

The diagnosis method for an autoimmune disease according to the present embodiment can be understood as a data collecting method for determining the presence or absence of contraction or a risk of contraction of the autoimmune disease, the method including a step of measuring the relative abundance of a bacterium included in a fecal sample collected from a test subject, in which the bacterium is a bacterium whose nucleotide sequence of 16S ribosomal RNA gene has an identity of 99% or higher with the nucleotide sequence set forth in any one of SEQ ID NO:3 to SEQ ID NO:23.

The diagnosis method for an autoimmune disease according to the present embodiment can also be understood as a data collecting method for determining a therapeutic effect for the autoimmune disease, the method including a step of measuring the relative abundance of a bacterium included in a fecal sample collected from a test subject, in which the bacterium is a bacterium whose nucleotide sequence of 16S ribosomal RNA gene has an identity of 99% or higher with the nucleotide sequence set forth in any one of SEQ ID NO:3 to SEQ ID NO:23.

### (Diagnosis program and diagnosis system for autoimmune disease)

The diagnosis method according to the invention as described above can also be provided as a program causing a computer to function as a diagnosis system for an autoimmune disease.

The program according to the present embodiment causes a computer to execute the following steps: a step of obtaining nucleotide sequence data by comprehensively decoding the nucleotide sequences of 16S ribosomal RNA gene of bacteria included in a fecal sample collected from a test subject; a step of calculating the frequency of the nucleotide sequence having an identity of 99% or higher with the nucleotide sequence set forth in any one of SEQ ID NO:3 to SEQ ID NO:23 from the nucleotide sequence data thus obtained, and calculating the relative abundance of the nucleotide sequence; a step of comparing the relative abundance thus calculated with a reference that has been inputted in advance, and determining the disease state of the autoimmune disease; and a step of outputting the determination result thus obtained.

According to the first embodiment, in a case in which the reference value is the relative abundance of a corresponding nucleotide sequence in healthy subject, the above-mentioned relative abundance is the relative abundance of a nucleotide sequence having an identity of 99% or higher with the nucleotide sequence set forth in SEQ ID NO:3 or SEQ ID NO:4, and the relative abundance thus calculated is large compared to the relative abundance in healthy subject, or in a case in which the relative abundance is the relative abundance of a nucleotide sequence having an identity of 99% or higher with the nucleotide sequence set forth in SEQ ID NO:5 to SEQ ID NO:23, and the relative abundance thus calculated is small compared to the relative abundance in healthy subject, it is determined that the test subject has contracted, or has a high risk of contracting, the autoimmune disease.

That is, the program according to the first embodiment causes a computer to execute the following steps: a step of obtaining nucleotide sequence data by comprehensively decoding the nucleotide sequences of 16S ribosomal RNA gene of bacteria included in a fecal sample collected from a test subject; a step of calculating the frequency of the nucleotide sequence having an identity of 99% or higher with the nucleotide sequence set forth in any one of SEQ ID NO:3 to SEQ ID NO:23 from the nucleotide sequence data thus obtained, and calculating the relative abundance of the nucleotide sequence; a step of comparing the relative abundance thus calculated with the relative abundance in healthy subject, which has been inputted in advance; a step of determining whether the test subject has contracted, or has a high risk of contracting, the autoimmune disease based on the comparison result; and a step of outputting the determination result thus obtained. In the determining step, in a case in which the above-mentioned relative abundance is the relative abundance of a nucleotide sequence having an identity of 99% or higher with the nucleotide sequence set forth in SEQ ID NO:3 or SEQ ID NO:4, and the relative abundance thus calculated is large compared to the relative abundance in healthy subject, or in a case in which the above-mentioned relative abundance is the relative abundance of a nucleotide sequence having an identity of 99% or higher with the nucleotide sequences set forth in SEQ ID NO:5 to SEQ ID NO:23, and the relative abundance thus calculated is small compared to the relative abundance in healthy subject, it is determined that the test subject has contracted, or has a high risk of contracting, the autoimmune disease.

The diagnosis system according to the first embodiment includes an input means for obtaining nucleotide sequence data by comprehensively decoding the nucleotide sequences of 16S ribosomal RNA gene of bacteria included in a fecal sample collected from a test subject; a calculation means for determining whether the test subject has contracted, or has a high risk of contracting, an autoimmune disease based on the nucleotide sequence data thus obtained; and an output means for outputting the determination result obtained by the calculation means.

The input means is a means for inputting comprehensively decoded nucleotide sequence data into a computer, and examples include various interfaces such as a mouse, a keyboard, a data transmission line, and a modem.

The calculation means (for example, CPU) executes a step of calculating the relative abundance from the appearance frequency of at least one nucleotide sequence having an identity of 99% or higher with the nucleotide sequences set forth in SEQ ID NO:3 to SEQ ID NO:23 from the inputted nucleotide sequence data; and a step of comparing the relative abundance thus calculated with a reference (that relative abundance in healthy subject) read from a storage device (for example, ROM or RAM); and (i) in a case in which the relative abundance is the relative abundance of a nucleotide sequence having an identity of 99% or higher with the nucleotide sequence set forth in SEQ ID NO:3 or SEQ ID NO:4, and the relative abundance in the test subject is large compared to the relative abundance in healthy subject, or (ii) in a case in which the relative abundance is the relative abundance of a nucleotide sequence having an identity of 99% or higher with the nucleotide sequences set forth in SEQ ID NO:5 to SEQ ID NO:23, and the relative abundance in the test subject is small compared to the relative abundance in healthy subject, determining that the test subject has contracted, or has a high risk of contracting, an autoimmune disease. In a case in which the condition does not conform to (i) or (ii), the calculation means may execute a step of determining that the test subject has not contracted, or does not have a high risk of contracting, an autoimmune disease.

The determination result is outputted into an output means such as, for example, a display or a printer. The determination result may also be outputted into another information processing terminal via a data transmission line or the like.

According to the second embodiment, in a case in which the test subject is a test subject before treatment of an autoimmune disease; the reference value is the relative abundance of a corresponding nucleotide sequence in the test subject before the treatment; and the relative abundance is the relative abundance of a nucleotide sequence having an identity of 99% or higher with the nucleotide sequence set forth in SEQ ID NO:3 or SEQ ID NO:4, and the relative abundance thus calculated is large compared to the relative abundance in the test subject before treatment, or in a case where in which the relative abundance is the relative abundance of a nucleotide sequence having an identity of 99% or higher with the nucleotide sequences set forth in SEQ ID NO:5 to SEQ ID NO:23, and the relative abundance thus calculated is small compared to the relative abundance in the test subject before treatment, it is determined that the disease state of the autoimmune disease of the test subject has been ameliorated by the treatment.

That is, the program according to the second embodiment causes a computer to execute the following steps: a step of obtaining nucleotide sequence data by comprehensively decoding the nucleotide sequences of 16S ribosomal RNA gene of bacteria included in a fecal sample collected from a test subject after treatment; a step of calculating the frequency of a nucleotide sequence having an identity of 99% or higher with the nucleotide sequence set forth in any one of SEQ ID NO:3 to SEQ ID NO:23 from the nucleotide sequence data thus obtained, and calculating the relative abundance of the nucleotide sequence; a step of comparing the relative abundance thus calculated with the relative abundance in the test subject before treatment, which has been inputted in advance; a step of determining, based on the comparison result, whether the disease state of the autoimmune disease of the test subject has been ameliorated by treatment; and a step of outputting the determination result thus obtained. In the determining step, in a case in which the relative abundance is the relative abundance of a nucleotide sequence having an identity of 99% or higher with the nucleotide sequence set forth in SEQ ID NO:3 or SEQ ID NO:4, and the relative abundance thus calculated is large compared to the relative abundance in the test subject before treatment, or in a case in which the relative abundance is the relative abundance of a nucleotide sequence having an identity of 99% or higher with the nucleotide sequences set forth in SEQ ID NO:5 to SEQ ID NO:23, and the relative abundance thus calculated is small compared to the relative abundance in the test subject before treatment, it is determined that the disease state of the autoimmune disease of the test subject has been ameliorated by the treatment.

The diagnosis system according to the second embodiment includes an input means for obtaining nucleotide sequence data by comprehensively decoding the nucleotide sequences of 16S ribosomal RNA gene of bacteria included in a fecal sample collected from a test subject after treatment; a calculation means for determining, based on the nucleotide sequence data thus obtained, whether the disease state of an autoimmune disease of the test subject has been ameliorated by the treatment; and an output means for outputting the determination result obtained by the calculation means.

According to the second embodiment, the calculation means (for example, CPU) executes a step of calculating the relative abundance from the appearance frequency of at least one nucleotide sequence having an identity of 99% or higher with the nucleotide sequences set forth in SEQ ID NO:3 to SEQ ID NO:23 from the inputted nucleotide sequence data; a step of comparing the relative abundance thus calculated with the reference value (above-mentioned relative abundance in the test subject before treatment) read from a storage device (for example, ROM or RAM), and (iii) in a case in which the relative abundance is the relative abundance of a nucleotide sequence having an identity of 99% or higher with the nucleotide sequence set forth in SEQ ID NO:3 or SEQ ID NO:4, and the relative abundance is large compared to the relative abundance in the test subject before treatment, or (iv) in a case in which the relative abundance is the relative abundance of a nucleotide sequence having an identity of 99% or higher with the nucleotide sequences set forth in SEQ ID NO:5 to SEQ ID NO:23, and the relative abundance is small compared to the relative abundance in the test subject before treatment, determining that the disease state of the autoimmune disease of the test subject has been ameliorated by the treatment. In a case in which the condition does not conform to (iii) or (iv), the calculation means may execute a step of determining that the disease state of the autoimmune disease of the test subject has not been ameliorated by the treatment.

In the second embodiment, in a case in which the test subject is a test subject after treatment of an autoimmune disease; the reference value is the relative abundance of the corresponding nucleotide sequence in the test subject after the treatment; and the relative abundance is the relative abundance of a nucleotide sequence having an identity of 99% or higher with the nucleotide sequence set forth in SEQ ID NO:3 or SEQ ID NO:4, and the relative abundance thus calculated is small compared to the relative abundance in the test subject after treatment, or in a case in which the relative abundance is the relative abundance of a nucleotide sequence having an identity of 99% or higher with the nucleotide sequences set forth in SEQ ID NO:5 to SEQ ID NO:23, while the relative abundance thus calculated is large compared to the relative abundance in the test subject after treatment, it may be determined that the disease state of the autoimmune disease of the test subject has been ameliorated by the treatment.

The program according to the present embodiment may be stored in a computer-readable recording medium. That is, the computer-readable recording medium according to the present embodiment has the above-described program recorded therein. The recording medium may be a non-transitory recording medium. Examples of the computer-readable recording medium include ROM or a hard disk of a computer; an external storage device installed in a server computer connected to the network; and portable recording media such as a flexible disk, a memory card, and an optical magnetic disk.

### [Biomarker for autoimmune disease diagnosis and use thereof]

The biomarker for autoimmune disease diagnosis according to the present embodiment comprises an intestinal bacterium whose nucleotide sequence of 16S ribosomal RNA gene has an identity of 99% or higher with any one of the nucleotide sequences set forth in SEQ ID NO:3 to SEQ ID NO:23.

As described above, in regard to an intestinal bacterium whose nucleotide sequence of the V1 region-V2 region of 16S rRNA gene is a nucleotide sequence set forth in SEQ ID NO:3 or 4, the relative abundance significantly increases in an MS patient compared to a healthy control. Furthermore, in an intestinal bacterium whose nucleotide sequence of the V1 region-V2 region of 16S rRNA gene is a nucleotide sequence set forth in any one of SEQ ID NO:5 to SEQ ID NO:23, the relative abundance significantly decreases in an MS patient compared to a healthy control. Therefore, the intestinal bacterium can be used as a biomarker based on the quantity of the relative abundance. When the biomarker of the present embodiment is used, for example, an autoimmune disease can be diagnosed by determining the presence or absence of contraction, or the risk of contraction, of an autoimmune disease, and determining the therapeutic effect of an autoimmune disease.

### [Preventing or treating agent for autoimmune disease]

The preventing or treating agent for an autoimmune disease according to the present embodiment contains, as an active ingredient, at least one selected from the group consisting of a bacterium whose nucleotide sequence of 16S ribosomal RNA gene has an identity of 99% or higher with any one of the nucleotide sequences set forth in SEQ ID NO:5 to SEQ ID NO:23, and a physiologically active substance derived from the bacterium.

As described above, in regard to an intestinal bacterium whose nucleotide sequence of the V1 region-V2 region of 16S rRNA gene is a nucleotide sequence set forth in any one of SEQ ID NO:5 to SEQ ID NO:23, the relative abundance significantly decreases in an MS patient compared to a healthy control. Since the prophylactic agent or treating agent according to the present embodiment contains this intestinal bacterium or a physiologically active substance derived from this, the preventing or treating agent is suitable for the prevention or treatment of an autoimmune disease such as MS (amelioration, alleviation, and remission of the disease state).

The intestinal bacterium as an active ingredient can be obtained by, for example, isolating and culturing intestinal bacteria that constitute the human intestinal bacterial flora, analyzing the nucleotide sequences of the V1 region-V2 region of 16S rRNA gene of the isolated intestinal bacteria, and specifying an intestinal bacterium having a desired nucleotide sequence. Furthermore, since an intestinal bacterium having a degree of similarity of 99% or higher with existing bacterial species is of the same kind as the bacterial species, the bacterial species may be purchased from a cell bank such as ATCC. A physiologically active substance derived from an intestinal bacterium can be obtained by culturing the intestinal bacterium and purifying or isolating the physiologically active substance secreted into the incubator. Furthermore, the physiologically active substance can also be obtained by purifying or isolating the substance from the intestinal tract contents of an animal such as a mouse, in which the intestinal bacterium has been inoculated and fixed (in vivo method).

The preventing or treating agent according to the present embodiment may be composed only of an active ingredient, or may further include pharmacologically acceptable carriers (an excipient, a binder, a disintegrant, a filler, an emulsifier, a flow additive regulating agent, and the like), or additives (a tonicity adjusting agent, a lubricating agent, a corrigent, a solubilizing agent, a suspending agent, a diluents, a surfactant, a stabilizer, an absorption promoter, an extending agent, a pH adjusting agent, a humectants, an adsorbent, a disintegration inhibitor, a coating agent, a colorant, a preservative, an antioxidant, fragrance, a flavoring agent, a sweetener, a buffering agent, a soothing agent, and the like).

The dosage form of the preventing or treating agent according to the present embodiment may be selected as appropriate according to the method of administration and the prescription conditions. Examples of the dosage form include a tablet, a pill, a granular preparation, a powder preparation, a capsule, a drop, a sublingual agent, a troche, and a liquid preparation. Furthermore, from the viewpoint of efficiently delivering the active ingredient to the large intestine, the preparation may be provided with an enteric coating. Regarding the enteric coating, any known enteric coating can be used without particular limitations.

The method for administering the preventing or treating agent according to the present embodiment may be any of oral administration and parenteral administration. In the case of parenteral administration, the preventing or treating agent may be administered directly into the intestinal tract.

Regarding the amount of administration of the preventing or treating agent according to the present embodiment, for example, in the case of administering the agent to a human male adult (bodyweight 60 kg), the amount of administration is usually 0.001 mg to 5,000 mg/day/person, and preferably 0.01 mg to 500 mg/day/person, in terms of the amount of the active ingredient. The preventing or treating agent may be administered in several divided portions.

### Examples

Hereinafter, the present invention will be explained more specifically based on Examples. However, the present invention is not intended to be limited to the following Examples.

### 1. Assay Method

### [1. Subjects]

Twenty MS patients (average age: 36.0 ± 7.2 years old, 6 males, and 14 females) and fifty healthy controls (HC) (average age: 27.2 ± 9.2 years old, 23 males and 27 females) were selected as subjects. The subjects were diagnosed according to McDonald's diagnosis criteria, and as a result, all of the MS patients were relapsing-remitting MS (RRMS) patients. Also, all of the MS patients did not develop any of primary progressive MS, secondary progressive MS, and other diseases. All of the subjects including the MS patients and healthy controls did not need to be administered with antibiotic agents while fecal samples were collected. The present assay was carried out according to the protocol acknowledged by the various committees on human research ethics of the National Center of Neurology and Psychiatry, Juntendo University Hospital, Azabu University Hospital, and the University of Tokyo Hospital. Informed consent was obtained in advance from all the subjects.

### [2. Collection and treatment of fecal samples]

Feces collected from the subjects were immediately put into disposable plastic bags containing an oxygen absorber and a carbon dioxide generating agent (the inside of the plastic bag is an environment in which oxygen-sensitive anaerobic bacteria can survive), and the plastic bags were transported to the laboratory while the plastic bags were maintained at a temperature of 4°C. In the laboratory, feces were suspended in phosphate-buffered physiological saline containing 20% glycerol, and the suspensions were immediately frozen with liquid nitrogen. The frozen suspensions were stored at -80°C until use.

Bacterial DNA's were isolated and purified from the fecal samples by the enzymatic degradation method described in a non-patent literature (DNA Res., 2013, Vol. 20, pp. 241-253).

Among the fifty healthy controls, fecal samples of forty healthy controls (HC40 group, average age: 28.5 ± 9.8 years old) were submitted to a test of comparison with fecal samples of twenty MS patients (MS20 group). In the comparison test, an evaluation of the differences between the compositions of the bacterial florae of the HC40 group and the MS20 group and identification of bacterial species having different existence ratios were conducted.

Eighteen healthy controls (long-term HC18 group, age: 21.9 ± 3.1 years old) were grouped as long-term observed HC18 group. Among the eighteen healthy controls, eight people were the subjects who were also in the HC40 group. From the eighteen subjects, fecal samples were collected nine times, once in every two weeks. Specifically, nine fecal samples were obtained from fourteen subjects, and eight fecal samples were obtained from four subjects. Bacterial species whose relative abundances are statistically significantly different between HC40 group and MS20 group were further evaluated using the fecal samples obtained from the long-term HC18 group, and it was evaluated whether the differences in the existence ratio along with the lapse of time were consistent.

Detailed data for the MS patients were as described in Table 1. Detailed data for the healthy controls were as described in Table 2.

**[Table 1]**

| MS patient ID | Gender | Age | Duration (years) | Relapse frequency (times/year) | Anti-AQP4 antibody in blood plasma | Treatment | Site of onset |
|---|---|---|---|---|---|---|---|
| Yms01 | Female | 36 | 15 | 2 | - | IFNβ1b+PSL | Cerebrum, medulla oblongata |
| Yms02 | Female | 40 | 24 | 1 | - | None | Cerebrum, cerebellum, brain stem, medulla oblongata |
| Yms04 | Female | 45 | 8 | 0 | - | None | Cerebrum, optic nerve |
| Yms05 | Male | 25 | 4 | 1 | - | IFNβ1a | Cerebrum, brain stem, medulla oblongata |
| Yms07 | Male | 41 | 7 | 0 | - | PSL | Cerebrum, brain stem, medulla oblongata |
| Yms08 | Female | 33 | 7 | 0 | - | IFNβ1b | Cerebrum, brain stem |
| Yms09 | Female | 19 | 3 | 1 | - | PSL | Cerebrum, brain stem, medulla oblongata, optic nerve |
| Yms10 | Male | 43 | 5 | 2 | - | PSL | Cerebrum, medulla oblongata |
| Yms11 | Female | 43 | 9 | 2 | - | None | Cerebrum, medulla oblongata |
| Yms12 | Female | 33 | 7 | 0 | - | None | Cerebrum |
| Yms14 | Male | 35 | 2 | 0 | - | None | Cerebrum |
| Yms15 | Female | 31 | 6 | 1 | NE | None | Cerebrum, medulla oblongata, optic nerve |
| Yms18 | Female | 27 | 10 | 1 | NE | PSL | Cerebrum, cerebellum, brain stem, medulla oblongata, optic nerve |
| Yms21 | Female | 33 | 7 | 0 | NE | IFNβ1a | Cerebrum, medulla oblongata, optic nerve |
| Yms23 | Female | 44 | 5 | 0 | - | IFNβ1a | Cerebrum, brain stem, optic nerve |
| Yms24 | Female | 27 | 3 | 0 | - | IFNβ1b | Cerebrum, brain stem, medulla oblongata |
| Yms26 | Female | 40 | 19 | 1 | - | IFNp1b | Cerebrum, medulla oblongata |
| Yms31 | Female | 42 | 4 | 0 | - | None | Cerebrum, optic nerve |
| Yms33 | Male | 44 | 20 | 0 | - | IFNβ1a | Cerebrum, brain stem, medulla oblongata |
| Yms34 | Male | 38 | 10 | 0 | - | IFNβ1a | Cerebrum, cerebellum, brain stem |

| | | | | | | | |
|---|---|---|---|---|---|---|---|
| PSL: Prednisolone, IFN: Interferon, NE: Not examined | | | | | | | |

**[Table 2]**

| Health control ID | Gender | Age | HC40 group | HC18 group |
|---|---|---|---|---|
| Apr10S00 | Female | 21 | ○ | - |
| APr14S00 | Female | 21 | ○ | - |
| APr15S00 | Female | 22 | ○ | - |
| APr17S00 | Male | 20 | ○ | ○ |
| APr19S00 | Male | 20 | ○ | ○ |
| APr20S00 | Female | 21 | ○ | ○ |
| APr21S00 | Female | 21 | ○ | - |
| APr22S00 | Female | 22 | ○ | ○ |
| APr23S00 | Female | 21 | ○ | ○ |
| APr24S00 | Male | 19 | ○ | - |
| APr27S00 | Female | 20 | ○ | - |
| APr30S00 | Female | 21 | ○ | - |
| APr31S00 | Male | 33 | ○ | ○ |
| APr35S00 | Female | 19 | ○ | - |
| APr36S00 | Female | 19 | ○ | - |
| APr37S00 | Female | 21 | ○ | ○ |
| APr40S00 | Male | 19 | ○ | ○ |
| F-AKO03 | Male | 50 | ○ | - |
| F-AKO05 | Male | 50 | ○ | - |
| F-AKO10 | Female | 28 | ○ | - |
| F-AKO17 | Female | 39 | ○ | - |
| F-AKO18 | Female | 33 | ○ | - |
| F-AKO23 | Male | 45 | ○ | - |
| F-AKO24 | Male | 35 | ○ | - |
| F-AKO27 | Male | 50 | ○ | - |
| F-BANK07 | Male | 31 | ○ | - |
| F-BANK08 | Male | 29 | ○ | - |
| F-BANK09 | Male | 28 | ○ | - |
| F-BANK10 | Male | 28 | ○ | - |
| F-Morita01 | Female | 23 | ○ | - |
| F-Morita02 | Female | 22 | ○ | - |
| F-Morita03 | Male | 23 | ○ | - |
| F-Morita04 | Male | 22 | ○ | - |
| F-Morita11 | Female | 22 | ○ | - |
| F-Morita21 | Male | 49 | ○ | - |
| F-Tagent06 | Male | 41 | ○ | - |
| F-Tagent15 | Male | 37 | ○ | - |
| F-Tagent16 | Female | 34 | ○ | - |
| F-Tagent17 | Male | 26 | ○ | - |
| F-Tegent18 | Female | 36 | ○ | - |
| Apr01S00 | Female | 21 | - | ○ |
| APr02S00 | Female | 23 | - | ○ |
| APr03S00 | Female | 21 | - | ○ |
| APr09S00 | Female | 20 | - | ○ |
| Apr11S00 | Female | 23 | - | ○ |
| APr12S00 | Male | 25 | - | ○ |
| APr16S00 | Female | 20 | - | ○ |
| APr29S00 | Female | 23 | - | ○ |
| APr32S00 | Male | 19 | - | ○ |
| APr39S00 | Male | 23 | - | ○ |

### [3. Determination of nucleotide sequences of V1-V2 region of 16S rRNA gene]

The VI-V2 region of 16S rRNA gene was amplified by PCR using forward primer 27Fmod (including a barcode sequence, SEQ ID NO:1: 5'-agrgtttgatymtggctcag-3') and reverse primer 338R (SEQ ID NO:2: 5'-tgctgcctcccgtaggagt-3'). In the presence of 250 µM dNTPs and 1U Ex Taq polymerase (manufactured by Takara Bio, Inc.), PCR was performed using a 1×Ex Taq PCR buffer (50 µL) containing 10 mM Tris-HCl (pH 8.3), 50 mM KCl, 1.5 mM MgCl₂, forward primer (0.2 µM), reverse primer (0.2 µM), and template DNA (< 20 ng). Regarding PCR, initial denaturation (96°C, for 2 minutes) was carried out using 9700 PCR System (manufactured by Life Technologies Japan, Ltd.), and then 25 cycles of denaturation (96°C, for 30 seconds), annealing (55°C, for 45 seconds), and elongation (72°C, for 1 minute) were repeated. Thus, PCR was carried out the final elongation (72°C, for 1 minute).

The PCR amplification product was purified using AMPure XP Magnetic purification beads (manufactured by Beckman Coulter, Inc.), and the purification product was quantitatively analyzed using Quant-iT PicoGreen dsDNA Assay Kit (manufactured by Life Technologies Japan, Ltd.). Various PCR amplification products were mixed such that the amount of the PCR amplification products would be equal amounts. The nucleotide sequences were determined using 454 GS FLX Titanium or 454 GS JUNIOR platform (manufactured by Roche Applied Science) according to the protocol described in the manual.

### [4. Establishment of full-length sequence database of 16S rRNA gene]

The full-length sequence database of 16S rRNA gene was established from the nucleotide sequence of the full-length 16S rRNA gene (FL-16S) registered in databases of RDP (http://rdp.cme.msu.edu/), CORE (http://microbiome.osu.edu/), and NCBI (http://www.ncbi.nlm.nih.gov/).

First, from the nucleotide sequences registered in the above-mentioned databases (total number of sequences: 221,537), nucleotide sequences having a sequence length of less than 1,400 base pairs, nucleotide sequences including 4 or more ambiguous bases, and nucleotide sequences suspected to be derived from eukaryotes were excluded (quality check), and high-quality FL-16S sequences (total number of sequences: 154,850) were obtained.

The high-quality FL-16S sequences thus obtained were subjected to clustering using USEARCH5 (threshold: identity of 99.8%), and 87,558 clusters corresponding to non-overlapping FL-16S sequences. These were designated as the full-length sequence database of FL-16S used for an analysis of the nucleotide sequences of the VI-V2 region of 16S rRNA gene.

### [5. Analysis of nucleotide sequence of V1-V2 region of 16S rRNA gene]

### (1) Estimation of assigned taxonomic groups

The bacterial florae of various samples were analyzed using the established analysis pipeline of read data (reads) of the nucleotide sequences of the V1-V2 region of 16S rRNA gene (see DNA Res., 2013, Vol. 20, pp. 241-253; and DNA Res., 2014, Vol. 21, pp. 15-25).

Briefly speaking, first, for the various samples, 3,000 units of 16S reads (average quality value > 25) were randomly selected from all the reads that had passed the quality check mentioned above. Primer sequences were eliminated from the selected 16S reads, and the resultants were used for the subsequent analyses. For the various samples, 3,000 units of 16S reads were subjected to clustering (threshold: identity of 96%), and the numbers of operational taxonomic units (OTU) were obtained. The diversity and richness of the bacterial species were evaluated using the numbers of OTU.

Next, the 16S reads were mapped using the full-length sequence database of FL-16S. Specifically, a BLAST analysis of the 16S reads (identity of ≥ 96%, coverage of ≥ 90%) was performed for the full-length sequence database of FL-16S (including 87,558 full-length nucleotide sequences corresponding to non-overlapping FL-16S sequences), and the 16S reads were mapped into FL-16S based on the analysis results. FL-16S sequences obtained by mapping the 16S reads were further subjected to clustering using USEARCH5 (threshold: identity of 97%), and thereby a 97% FL-16S cluster corresponding to OTU at the species level (hereinafter, also referred to as "rclust", and "rclust" was attached to the cluster name) was produced. The assigned taxonomic group of 16S reads was estimated at the species level based on the 97% FL-16S clusters for which the 16S reads had been mapped.

Regarding 16S reads that were not mapped, an OTU (hereinafter, also referred to as "unmap_OTU", and "unmap_OTU" was attached to the cluster name) was produced by standard clustering using USEARCH5 (threshold: identity of 96%). The assigned taxonomic groups of unmapped 16S reads were estimated to be of higher taxonomic levels (that is, genus, phylum, and the like), based on the identity search results for the full-length sequence database of FL-16S.

The bacterial florae were analyzed at the levels of species, genus, and phylum, from the numbers of 16S reads assigned to the "rclust" and "unmap_OTU". The nucleotide sequences of the V1-V2 region of 16S rRNA gene used in the analysis were registered in the DDBJ/GenBank/EMBL database under Accession Nos. DRA000672, DRA000673, DRA000675, DRA000676, DRA000678-DRA000684, DRA002866-DRA002874 (MS patients), and DRA002875-DRA002906 (healthy controls).

### (2) Analysis of degree of similarity of bacterial florae (Unifrac analysis)

The differences in the overall composition of the intestinal bacterial flora were analyzed by a UniFrac analysis. The richness of the OTU's of various samples based on the estimated amount of Chao1 was calculated using Vegan package (v. 2.0-5) mounted in R (version 2.15.2).

### (3) Statistical analysis

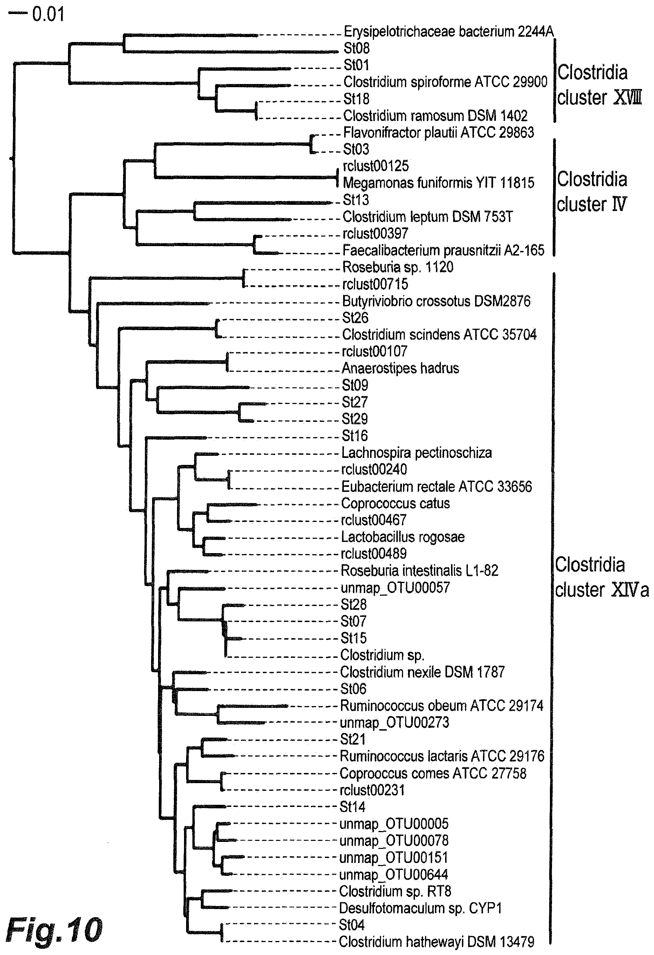

For all statistical analyses, R (version 2.15.2) was used. The richness, degree of uniformity, and diversity of bacterial species were evaluated using R Vegan package. The statistical test was conducted by Welch's t-test. Furthermore, the p-value of multiple test was corrected by the Benjamini-Hochberg method. The phylogenetic tree was produced by a neighbor joining method. The length of each node in the phylogenetic tree represents the probability evaluated by a bootstrap method (1,000 repetitions) (the length of "-" shown in the upper left corner of Fig. 10 corresponds to a probability of 0.01).

### 2. Results

### [1. Assignment of 16S reads]

From the fecal samples of MS20 group and HC40 group, high-quality 16S reads of 141,549 reads (7,080 ± 825 reads per sample) and 303,585 reads (7,590 ± 616 reads), respectively, were obtained using 454 GS FLX Titanium (see Table 3 and Table 4).

**[Table 3]**

| MS patient ID | Total number of reads | Number of reads that passed filter | Proportion of reads that passed filter (%) |
|---|---|---|---|
| Yms01 | 10211 | 4993 | 48.9 |
| Yms02 | 10403 | 4716 | 45.33 |
| Yms04 | 26291 | 12924 | 49.16 |
| Yms05 | 27087 | 14431 | 53.28 |
| Yms07 | 19862 | 5731 | 28.85 |
| Yms08 | 17813 | 4296 | 24.12 |
| Yms09 | 16588 | 4533 | 27.33 |
| Yms10 | 14888 | 8143 | 54.7 |
| Yms11 | 13549 | 8942 | 66 |
| Yms12 | 15428 | 8968 | 58.13 |
| Yms14 | 7527 | 4098 | 54.44 |
| Yms15 | 26546 | 16638 | 62.68 |
| Yms18 | 7626 | 4458 | 58.46 |
| Yms21 | 7827 | 3281 | 41.92 |
| Yms23 | 9820 | 5507 | 56.08 |
| Yms24 | 9636 | 5062 | 52.53 |
| Yms26 | 11952 | 5808 | 48.59 |
| Yms31 | 10055 | 6202 | 61.68 |
| Yms33 | 19174 | 7956 | 41.49 |
| Yms34 | 9568 | 4907 | 51.29 |
| Average | 14596 ± 1435 | 7080 ± 825 | 49.25 |

**[Table 4]**

| Healthy control ID | Total number of reads | Number of reads that passed filter | Proportion of reads that passed filter (%) |
|---|---|---|---|
| Apr10S00 | 10308 | 6167 | 59.83 |
| APr14S00 | 27106 | 15241 | 56.23 |
| APr15S00 | 21307 | 10771 | 50.55 |
| APr17S00 | 7434 | 4713 | 63.4 |
| APr19S00 | 16030 | 8601 | 53.66 |
| APr20S00 | 7682 | 4725 | 61.51 |
| APr21S00 | 27362 | 14996 | 54.81 |
| APr22S00 | 9606 | 5423 | 56.45 |
| APr23S00 | 8021 | 4748 | 59.19 |
| APr24S00 | 22540 | 12366 | 54.86 |
| APr27S00 | 29984 | 17304 | 57.71 |
| APr30S00 | 8531 | 5291 | 62.02 |
| APr31S00 | 9535 | 6394 | 67.06 |
| APr35S00 | 7041 | 4406 | 62.58 |
| APr36S00 | 15938 | 9589 | 60.16 |
| APr37S00 | 11054 | 6751 | 61.07 |
| APr40S00 | 6756 | 4139 | 61.26 |
| F-AK003 | 8252 | 4542 | 55.04 |
| F-AKO05 | 10282 | 4524 | 44 |
| F-AKO10 | 10611 | 5624 | 53 |
| F-AKO17 | 12829 | 7509 | 58.53 |
| F-AKO18 | 9015 | 4965 | 55.07 |
| F-AKO23 | 12987 | 7201 | 55.45 |
| F-AKO24 | 7273 | 3493 | 48.03 |
| F-AKO 27 | 12479 | 8591 | 68.84 |
| F-BANK07 | 10115 | 5288 | 52.28 |
| F-BANK08 | 10589 | 5530 | 5222 |
| F-BANK09 | 9643 | 5257 | 54.52 |
| F-BANK10 | 7368 | 4157 | 56.42 |
| F-Morita01 | 13770 | 8328 | 60.48 |
| F-Morita02 | 13115 | 8561 | 65.28 |
| F-Morita03 | 14178 | 8816 | 62.18 |
| F-Morita04 | 13917 | 8559 | 61.5 |
| F-Morita11 | 13991 | 9048 | 64.67 |
| F-Morita21 | 9700 | 5415 | 55.82 |
| F-Tagent06 | 33016 | 20302 | 61.49 |
| F-Tagent15 | 8594 | 4377 | 50.93 |
| F-Tagent16 | 22360 | 11630 | 52.01 |
| F-Tagent17 | 9341 | 5851 | 62.64 |
| F-Tagent18 | 7650 | 4392 | 57.41 |
| Average | 13183 ± 1071 | 7590 ± 616 | 57.75 |

For the full-length sequence database of FL-16S (including 87,558 full-length nucleotide sequences corresponding to non-overlapping FL-16S sequences), a BLAST analysis (identity of ≥ 96%, coverage of ≥ 90%) was performed using 3,000 reads randomly selected for each sample (total 180,000 reads), and as a result, 163,691 reads (HC40 group-derived: 109,891 reads, MS20 group-derived: 53,800 reads) were mapped into non-overlapping 9,816 clusters. On the other hand, the remaining 16,309 reads (HC40 group-derived: 10,109 reads, MS20 group-derived: 6,200 reads) were not mapped into any cluster. As a result, it was found that about 91% of all of the 16S reads can belong to known species or strains. The proportion of unmapped reads was 8.4% for the HC40 group and 10.3% for the MS20 group. From these, it was suggested that the proportion of bacteria that are unknown at the species level is slightly larger in the MS20 group than in the HC40 group.

FL-16S sequences resulting from mapping of 16S reads were further subjected to clustering using USEARCH5 (threshold: identity of 97%), and as a result, 760 clusters exhibiting similarity at the species level were produced. Among these clusters, clusters with an average relative abundance of less than 0.1% (659 clusters) were excluded from subsequent analyses. That is, 101 clusters were further provided for the analyses.

Standard clustering using USEARCH5 (threshold: identity of 96%) was performed for the unmapped 16S reads, and as a result, 1,321 OTU's were produced. Among these OTU's, OTU's with an average relative abundance of less than 0.1% (1,292 units) were excluded from subsequent analyses. That is, 29 OTU's were further provided for the analyses.

The 101 clusters and 29 OTU's that were further provided for the analyses included 163,726 reads (HC40 group-derived: 109,913 reads, MS20 group-derived: 53,813 reads). This number corresponds to about 91% of 180,000 reads (3,000 reads/test subject) initially used for the analysis.

### [2. Comparison of intestinal bacterial florae between MS patients and healthy controls]

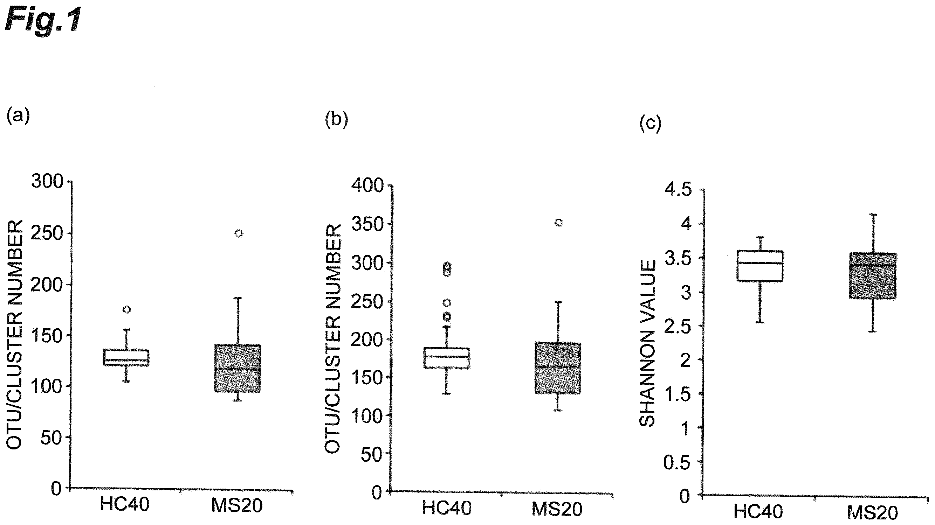

Fig. 1(a) is a graph showing the average values of the number of OTU's and clusters of MS20 group and HC40 group. The axis of ordinate represents the number of OTU's and clusters. Fig. 1(b) is a graph showing the Chaol estimates of the number of OTU's and clusters of MS20 group and HC40 group. The axis of ordinate represents the number of OTU's and clusters. Fig. 1(c) is a graph showing the Shannon values of MS20 group and HC40 group. The axis of ordinate represents the Shannon value.

The average value of the number of OTU's and clusters, and the Chao1 estimate of the MS20 group were 126.9 and 172.8, respectively. The average value of the number of OTU's and clusters, and the Chao1 estimate of the HC40 group were 129.4 and 184.8, respectively. The values were both slightly lower in the MS20 group than in the HC40 group; however, there were no statistically significant differences (Fig. 1(a) and Fig. 1(b)). Furthermore, the Shannon value, which is a diversity index reflecting the richness of species and the degree of uniformity, showed no meaningful difference between the MS20 group (3.29 ± 0.46) and the HC40 group (3.39 ± 0.29) (Fig. 1(c)).

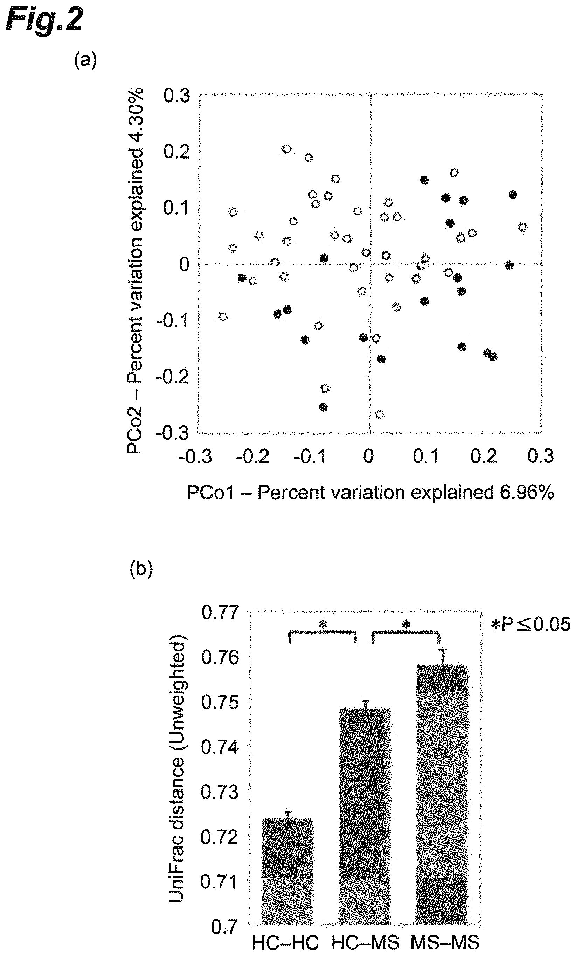

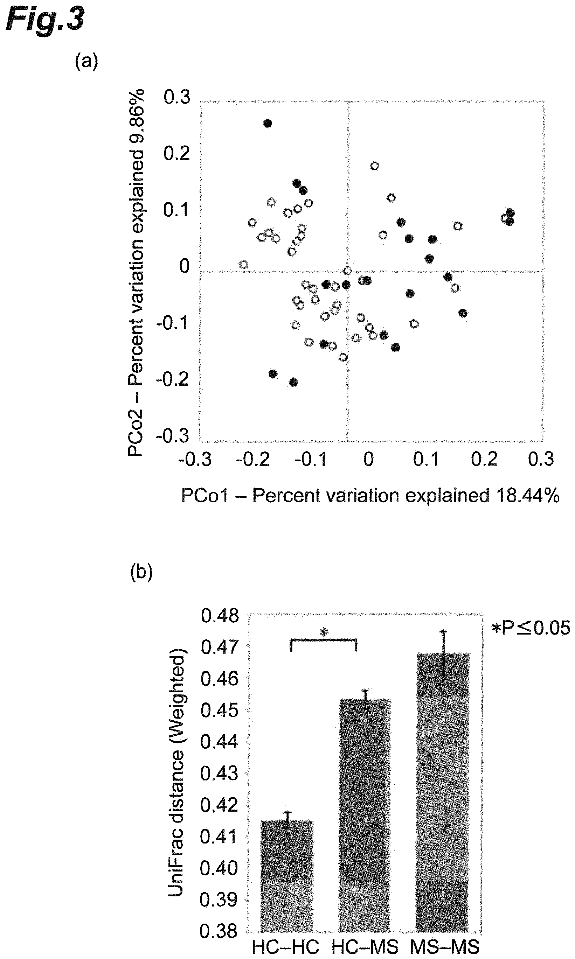

Fig. 2 and Fig. 3 are graphs showing the results of a UniFrac distance analysis (Fig. 2(b) and Fig. 3(b)) and a UniFrac principal coordinates analysis (PCoA) (Fig. 2(a) and Fig. 3(a)). Fig. 2 and Fig. 3 correspond to the results of unweighted and weighted UniFrac analyses, respectively. Open circles (○) and filled circles (●) in Fig. 2(a) and Fig. 3(a) correspond to the data of individual subjects of the HC40 group and the MS20 group, respectively. In Fig. 2(a), the results of a similarity matrix analysis (ANOSIM) were such that R = 0.239 and p ≤ 0.00009. In Fig. 3(a), the results of ANOSIM were such that R = 0.208 and p ≤ 0.002. The symbol "*" in Fig. 2(b) and Fig. 3(b) represents that p ≤ 0.05.

As shown in Fig. 2 and Fig. 3, for both of the unweighted and weighted UniFrac analyses, there was a significant difference (p < 0.05) between the compositions of the intestinal bacterial florae of the MS20 group and the HC40 group. Furthermore, the MS20 group showed large variations in the intestinal bacterial flora among subjects (individuals), compared to the HC40 group. From these results, it was suggested that dysbiosis occurred at a moderate level in the MS20 group, compared to the HC40 group.

### [3. Identification of bacterial species having differences in relative abundance between MS patients and healthy controls]

Next, in order to identify bacterial species whose relative abundance in the intestinal bacterial flora differs between the MS20 group and the HC40 group, the bacterial species compositions were analyzed at various taxonomic levels.

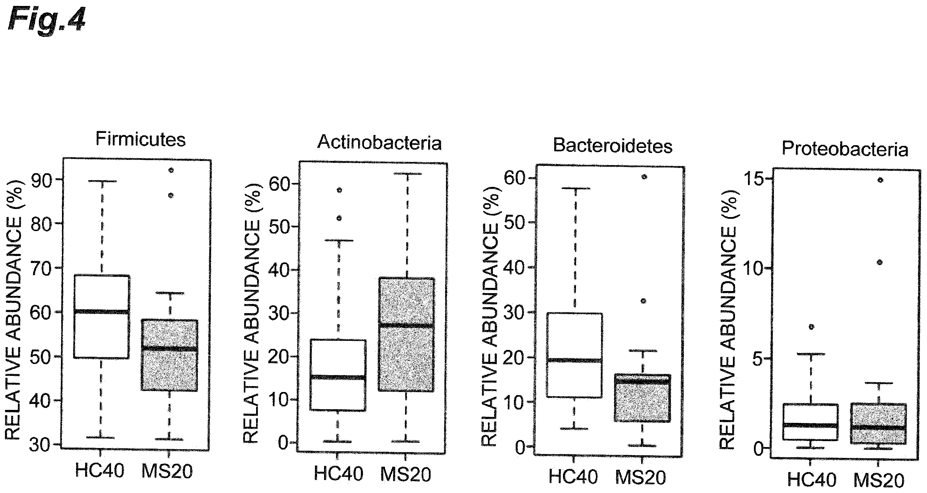

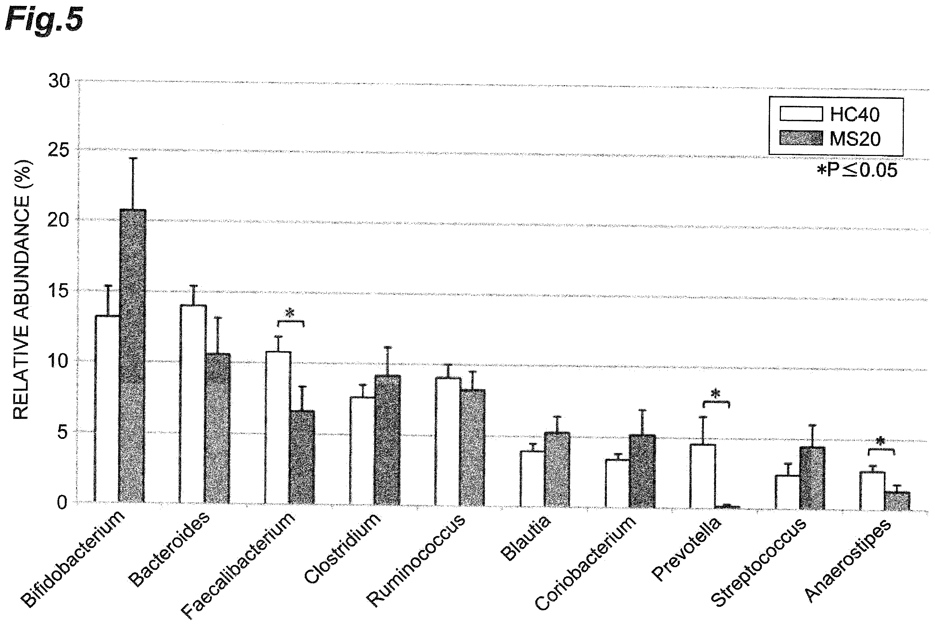

Fig. 4 is a graph showing the results of analyzing the bacterial species compositions in the intestinal bacterial florae of MS20 group and HC40 group at the phylum level. The axis of ordinate represents the relative abundance (%). Fig. 5 is a graph showing the results of analyzing the bacterial species compositions in the intestinal bacterial florae of the MS20 group and the HC40 group at the genus level. The axis of ordinate represents the relative abundance (%), open rods represent the HC40 group, and solid rods represent the MS20 group. The symbol "*" in the graph represents that p ≤ 0.05.

As a result of analyzing the intestinal bacterial florae at the phylum level, the intestinal bacterial florae of the MS20 group and the HC40 group were all composed of bacterial belonging to four major phyla (phylum Actinobacteria, phylum Bacteroidetes, phylum Firmicutes, and phylum Proteobacteria).

The MS20 group showed a tendency that the relative abundances of bacteria belonging to the phylum Actinobacteria were large, and the relative abundances of bacteria belonging to the phylum Firmicutes and the phylum Bacteroidetes were small, compared to the HC40 group; however, there were no statistically significant differences (Fig. 4).

As a result of an analysis at the genus level, the MS20 group showed a tendency that the relative abundances of bacteria belonging to the genus Bacteroides, genus Faecalibacterium, genus Prevotella, and genus Anaerostipes were small, and the relative abundances of bacteria belonging to the genus Bifidobacterium and the genus Streptococcus were large, compared to the HC40 group (Fig. 5). Particularly, there were statistically significant differences in the relative abundances of bacteria belonging to the genus Bacteroides, genus Prevotella, and genus Anaerostipes (Fig. 5).

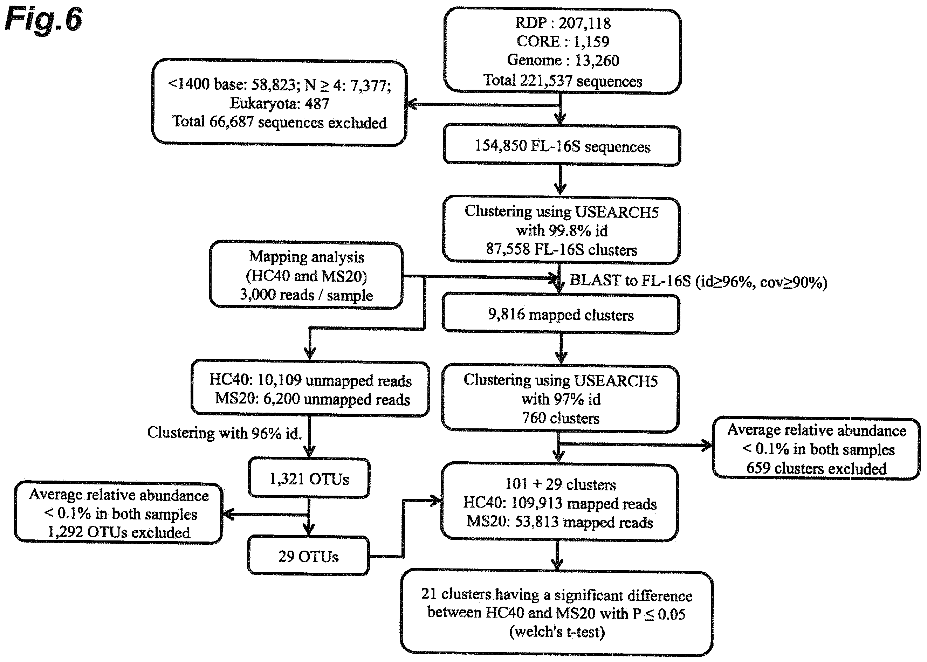

As a result of an analysis at the species level, 21 bacterial species showing statistically significant differences (p < 0.05) in the relative abundances of bacteria between the MS20 group and the HC40 group were identified (Table 5). Fig. 6 shows a workflow of the mapping analysis of 16S reads.

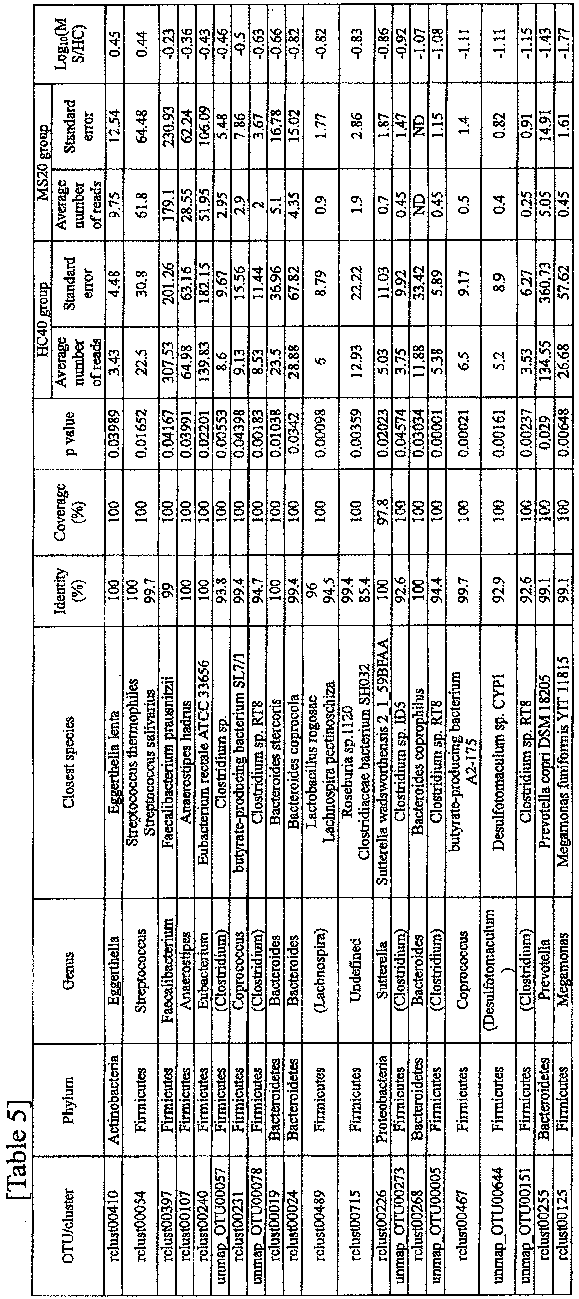

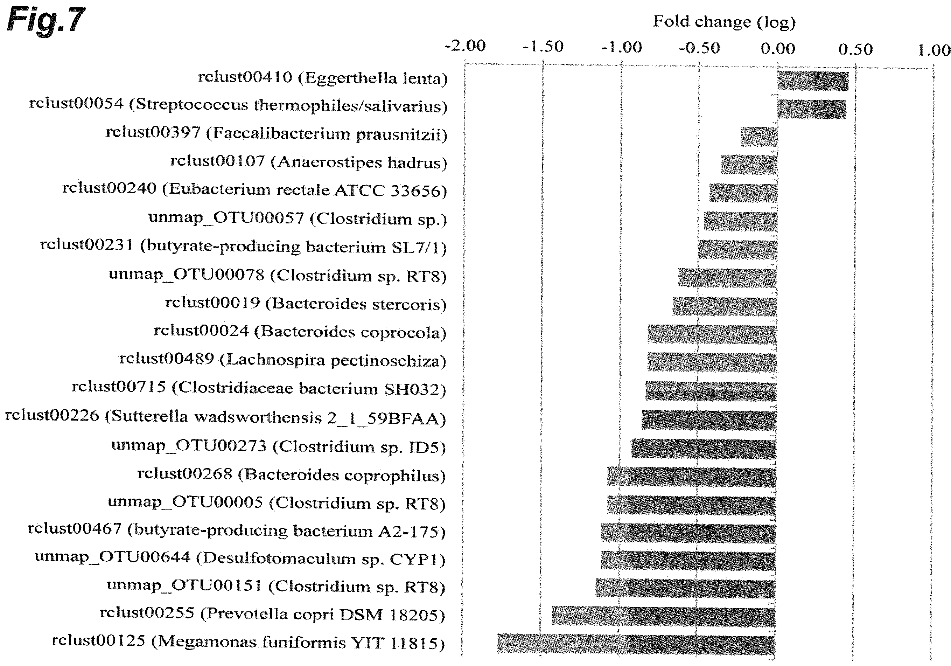

Fig. 7 is a graph showing the differences in the relative abundances of bacteria between the MS20 group and the HC40 group (Log₁₀(average number of reads of MS20 group /average number of reads of HC40 group)). The axis of ordinate in Fig. 7 represents the difference in the relative abundances of bacteria, and the differences in the relative abundances of bacteria correspond to the values of "Log₁₀(MS/HC)" in Table 5. In Fig. 7, the bacterial species indicated within parentheses are twenty-one bacterial species showing the highest degree of similarity to the respective representative nucleotide sequences of the V1-V2 region of 16S rRNA.

Among the twenty-one species thus identified, fifteen species were classified as "rclust", which showed an identity of 96% or higher with the known FL-16S nucleotide sequence. The other six species were classified as "unmap_OTU", which showed an identity of lower than 96% only with the known FL-16S nucleotide sequence (not mapped) (Table 5 and Fig. 7).

rclust00231 and rclust00467 both showed an identity of higher than 99% with a butyric acid-producing bacterium, the genus of which was not identified. In addition, since rclust00231 showed an identity of 97.4% with *Coprococcus comes* ATCC 27758 (Accession No.: NZ_ABVR00000000), and rclust00467 showed an identity of 95.2% with *Coprococcus catus* (Accession No.: S001014091), these species both belonged to the genus Coprococcus (Table 5).

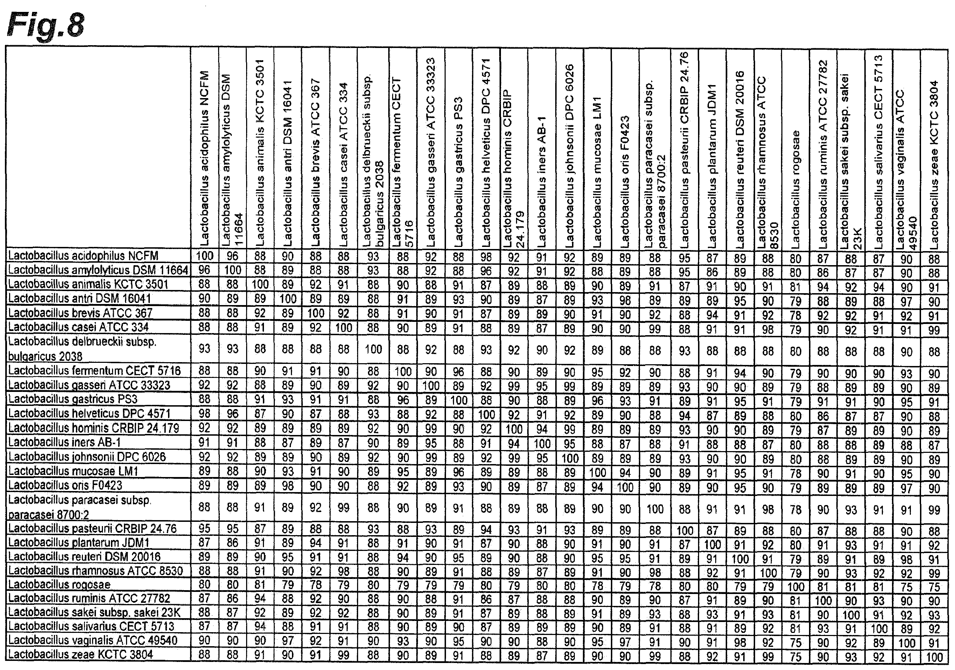

rclust00489 showed an identity of 96.0% with *Lactobacillus rogosae* (Accession No.: S001873784). However, regarding *Lactobacillus rogosae,* as a result of an analysis of the degree of similarity to the nucleotide sequences of the V1-V2 region of 16S rRNA of other known bacterial species of the genus Lactobacillus, there was found a possibility that *Lactobacillus rogosae* could be phylogenetically different from these bacterial species of the genus Lactobacillus (Fig. 8). Fig. 8 is a table showing the results of analyzing the degrees of similarity of nucleotide sequences of the V1-V2 region of 16S rRNA. The values in Fig. 8 represent the identity (%) of the nucleotide sequences of the V1-V2 region of 16S rRNA between the bacterial species shown at the top and the bacterial species shown in the left-hand side. As shown in Fig. 8, *Lactobacillus rogosae* showed an identity of 81% or lower only with other bacterial species of the genus Lactobacillus. Meanwhile, rclust00489 showed a high identity (94.5%) with *Lachnospira pectinoschiza* (as shown in Fig. 10, bacterial species belonging to Clostridium cluster XIVa). Also from the results of a phylogenetic analysis of the bacterial species of Clostridium species that will be described below, rclust00489 can be assigned to unidentified bacterial species belonging to Clostridium cluster XIVa.

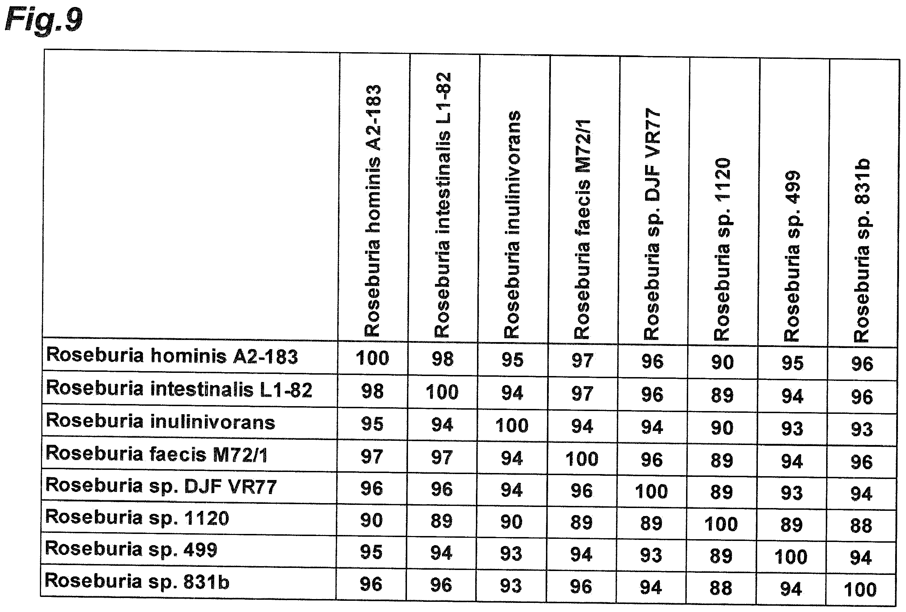

rclust00715 showed an identity of 99.4% with Roseburia sp. 1120 (Accession No.: S003610183). However, regarding Roseburia sp. 1120, as a result of an analysis of the degree of similarity of the nucleotide sequence of the V1-V2 region of 16S rRNA with other known bacterial species of the genus Roseburia, such as *Roseburia faecis, Roseburia intestinalis,* and *Roseburia hominis,* there was found a possibility that Roseburia sp. 1120 could be phylogenetically different from these bacterial species of the genus Roseburia (Fig. 9). Fig. 9 is a table showing the results of analyzing the degrees of similarity of nucleotide sequences of the V1-V2 region of 16S rRNA. The values in Fig. 9 represent the identity (%) of the nucleotide sequences of the V1-V2 region of 16S rRNA between the bacterial species shown at the top and the bacterial species shown on the left-hand side. As shown in Fig. 9, Roseburia sp. 1120 showed an identity of 90% or lower only with other bacterial species of the genus Roseburia. Meanwhile, rclust00715 showed the second highest identity (85.4%) with a bacterium belonging to the family Clostridiaceae, SH032 (Accession No.: S000994782). Also from the results of a phylogenetic analysis of the bacterial species of Clostridia that will be described below, rclust00715 can be assigned to unidentified bacterial species belonging to Clostridium cluster XIVa.

Furthermore, all of the six bacterial species classified as ""unmap_OTU" could not belong to known bacterial species at the species level and the genus level; however, from the results of a phylogenetic analysis of bacterial species of Clostridia that will be described below, the six bacterial species can all be assigned to the bacterial species belonging to Clostridium cluster XIVa.

Among the identified twenty-one species, two species (rclust00410 and rclust00054) showed large relative abundances in the MS20 group compared to the HC40 group, and the other nineteen species showed small relative abundances in the MS20 group compared to the HC40 group (Table 5 and Fig. 7).

Among the identified twenty-one species, four species belonged to the phylum Bacteroidetes, one species belonged to the phylum Actinobacteria, one species belonged to the phylum Proteobacteria, and fifteen species belonged to the phylum Firmicutes.

Among the fifteen species belonging to the phylum Firmicutes, fourteen species belonged to a clade (monophyletic group) of Clostridia. Thus, in order to determine more specific assigned taxonomic groups, a phylogenetic analysis based on the nucleotide sequence of the V1-V2 region of 16S rRNA was performed for these fourteen species and known bacterial species of Clostridia. The known bacterial species of Clostridia included seventeen bacterial species that had been found to induce Treg in the colon (see Non-Patent Literature 4. Hereinafter, also referred to as "St bacterial species").

Fig. 10 is a diagram showing the results of a phylogenetic analysis of bacterial species of Clostridia. In Fig. 10, the St bacterial species among the known bacterial species were assigned with "St" in front of the name. The fourteen species identified in the present example are indicated by their OTU names or cluster names. As shown in Fig. 8, among the fourteen species identified in the present example, twelve species belonged to Clostridium cluster XIVa, and two species belonged to Clostridium cluster IV.

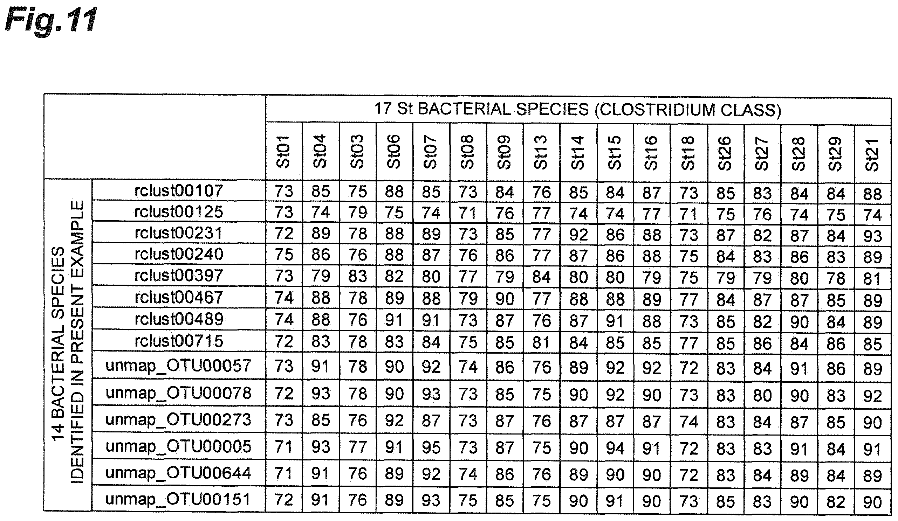

Furthermore, interestingly, the fourteen species identified in the present example did not include any species located close to the seventeen St bacterial species in the phylogenetic tree (Fig. 10). This was proved also from the results of an analysis of the degree of similarity (identity) of the nucleotide sequence of the V1-V2 region of 16S rRNA in these subsets (Fig. 11). Fig. 11 is a table showing the results of analyzing the degrees of similarity of the nucleotide sequences of the V1-V2 region of 16S rRNA. The values in Fig. 11 represent the identity (%) of the nucleotide sequences of the VI-V2 region of 16S rRNA between the bacterial species shown at the top and the bacterial species shown on the left-hand side. For example, the number 73 shown immediately below St01 represents that the identity of the nucleotide sequences of the V1-V2 region of 16S rRNA between St01 and rclust00107 is 73%. As shown in Fig. 11, the fourteen species identified in the present example and the seventeen St bacterial species were such that the identity of the nucleotide sequences of the VI-V2 region of 16S rRNA was 95% in all cases. Meanwhile, the fourteen species identified in the present example are all bacterial species with small relative abundances in the MS20 group.

The nucleotide sequences of the V1-V2 region of 16S rRNA of the identified twenty-one species are presented in Table 6 to Table 8.

**[Table 6]**

| SEQ ID NO: | OTU/cluster | Nucleotide sequence (5'→3') |
|---|---|---|
| 3 | rclust00410 | GATGAACGCTGGCGGCGTGCCTAACACATGCAAGTCGAACGATGAAACCGCCCTC GGGCGGACATGAAGTGGCGAACGGGTGAGTAACACGTGACCAACCTGCCCCCCTC TCCGGGACAACCTTGGGAAACCGAGGCTAATACCGGATACTCCCTCCCCTGCTCCT GCAGGGGTCGGGAAAGCCCAGGCGGAGGGGGATGGGGTCGCGGCCCATTAGGTA GTAGGCGGGGTAACGGCCCACCTAGCCCGCGATGGGTAGCCGGGTTGAGAGACC GACCGGCCACATTGGGACTGAGATACGGCCCAG |
| 4 | rclust00054 | GACGAACGCTGGCGGCGTGCCTAATACATGCAAGTAGAACGCTGAAGAGAGGAGC TTGCTCTTCTTGGATGAGTTGCGAACGGGTGAGTAACGCGTAGGTAACCTGCCTTG TAGCGGGGGATAACTATTGGAAACGATAGCTAATACCGCATAACAATGGATGACAC ATGTCATTTATTTGAAAGGGGCAATTGCTCCACTACAAGATGGACCTGCGTTGTATT AGCTAGTAGGTGAGGTAATGGCTCACCTAGGCGACGATACATAGCCGACCTGAGAG GGTGATCGGCCACACTGGGACTGAGACACGGCCCAG |
| 5 | rclust00397 | GACGAACGCTGGCGGCGCGCCTAACACATGCAAGTCGAACGAGAGATGAGGAGCT TGCTCTTCAAATCGAGTGGCGAACGGGTGAGTAACGCGTGAGGAACCTGCCTCAAA GAGGGGGACAACAGTTGGAAACGACTGCTAATACCGCATAAGCCCACGGCTCGGC ATCGAGCAGAGGAAAGGAGTGATCCGCTTTGAGATGGCCTCGCGTCCGATTAGCTA GTTGGTGAGGTAACGGCCCACCAAGGCGACGATCGGTAGCCGGACTGAGAGGTTG AACGGCCACATTGGGACTGAGACACGGCCCAG |
| 6 | rclust00107 | GATGAACGCTGGCGGCGTGCTTAACACATGCAAGTCGAACGAAACACCTTATTTGA TTTTCTTCGGAACTGAAGATTTGGTGATTGAGTGGCGGACGGGTGAGTAACGCGTG GGTAACCTGCCCTGTACAGGGGGATAACAGTCAGAAATGACTGCTAATACCGCATA AGACCACAGCACCGCATGGTGCAGGGGTAAAAACTCCGGTGGTACAGGATGGACC CGCGTCTGATTAGCTGGTTGGTGAGGTAACGGCTCACCAAGGCGACGATCAGTAGC CGGCTTGAGAGAGTGAACGGCCACATTGGGACTGAGACACGGCCCAA |
| 7 | rclust00240 | GATGAACGCTGGCGGCGTGCTTAACACATGCAAGTCGAACGAAGCACTTTATTTGA TTTCCTTCGGGACTGATTATTTTGTGACTGAGTGGCGGACGGGTGAGTAACGCGTG GGTAACCTGCCTTGTACAGGGGGATAACAGTTGGAAACGGCTGCTAATACCGCATA AGCGCACGGCATCGCATGATGCAGTGTGAAAAACTCCGGTGGTATAAGATGGACCC GCGTTGGATTAGCTAGTTGGTGAGGTAACGGCCCACCAAGGCGACGATCCATAGCC GACCTGAGAGGGTGACCGGCCACATTGGGACTGAGACACGGCCCAA |
| 8 | unmap_OTU00057 | GATGAACGCTGGCGGCGTGCTTAACACATGCAAGTCGAACGAAGCAATCTAACGGA AGTTTTCGGATGGAAGCTGGATTGACTGAGTGGCGGACGGGTGAGTAACGCGTGG GTAACCTGCCTCACACTGGGGGACAACAGTTAGAAATGACTGCTAATACCGCATAA GCGCACAGGACCGCATGGTCCGGTGTGAAAAACTCTAGTGGTGTGAGATGGACCC GCGTTTGATTAGCTAGTTGGTGGGGTAACGGCCTACCAAGGCGACGATCAATAGCC GACCTNAGAGGGTGACCGGCCACATTGGGACTGAGACACGGCCCAA |
| 9 | rclust00231 | GATGAACGCTGGCGGCGTGCTTAACACATGCAAGTCGAACGAAGCACTTATCTTTG AATCTTCGGATGAAGAGGTTTGTGACTGAGTGGCGGACGGGTGAGTAACGCGTGG GTAACCTGCCTCATACAGGGGGATAACAGTTAGAAATGACTGCTAATACCGCATAAG ACCACGGAGCCGCATGGCTCAGTGGGAAAAACTCCGGTGGTATGAGATGGACCCG CGTCTGATTAGGTAGTTGGTGGGGTAACGGCCTACCAAGCCAACGATCAGTAGCCG ACCTGAGAGGGTGACCGGCCACATTGGGACTGAGACACGGCCCAA |

**[Table 7]**

| SEQ ID NO: | OTU/cluster | Nucleotide sequence (5'→3') |
|---|---|---|
| 10 | unmap_OTU00078 | GATGAACGCTGGCGGCGTGCTTAACACATGCAAGTCGAGCGAAGCGATTTAAATGA GACTTCGGTGGATTTTAAATTGACTGAGCGGCGGACGGGTGAGTAACGCGTGGATA ACCTGCCTCACACAGGGGGATAACAGTTAGAAATGACTGCTAATACCGCATAAGCGC ACGGTACCGCATGGTACAGTGCGAAAAACTCCGGTGGTGTGAGATGGATCCGCGTC TGATTAGGTAGTTGGTGAGGTAACGGCCCACAAGCCGACGATCAGTAGCCGACCTG AGAGGTGACCGGCACATTGGGACTGAGACACGGCCCAG |
| 11 | rclust00019 | GATGAACGCTAGCTACAGGCTTAACACATGCAAGTCGAGGGGCAGCATCATCAAAG CTTGCTTTGATGGATGGCGACCGGCGCACGGGTGAGTAACACGTATCCAACCTGCCG ACAACACTGGGATAGCCTTTCGAAAGAAAGATTAATACCGGATGGCATAGTTTTCCC GCATGGGATAATTATTAAAGAATTTCGGTTGTCGATGGGGATGCGTTCCATTAGGCAG TTGGCGGGGTAACGGCCCACCAAACCAACGATGGATAGGGGTTCTGAGAGGAAGGT CCCCCACATTGGAACTGAGACACGGTCCAA |
| 12 | rclust00024 | GATGAACGCTAGCTACAGGCTTAACACATGCAAGTCGAGGGGCAGCATGAACTTAG CTTGCTAAGTTTGATGGCGACCGGCGCACGGGTGAGTAACACGTATCCAACCTTCCG TTTACTCAGGGATAGCCTTTCGAAAGAAAGATTAATACCTGATAGTATGGTGAGATTG CATGATAGCACCATTAAAGATTTATTGGTAAACGATGGGGATGCGTTCCATTAGGTAG TAGGCGGGGTAACGGCCCACCTAGCCGACGATGGATAGGGGTTCTGAGAGGAAGGT CCCCCACATTGGAACTGAGACACGGTCCAA |
| 13 | rclust00489 | GATGAACGCTGGCGGCGTGCTTAACACATGCAAGTCGAACGAAGCATTTAAGACGG ATTCTTTCGGGATGAAGACTTTTATGACTGAGTGGCGGACGGGTGAGTAACGCGTGG GTAACCTGCCTCACACAGGGGGATAGCAGTTGGAAACGGCTGATAATACCGCATAA GCGCACAGTACCGCATGGTACAGTGTGAAAAACTCCGGTGGTGTGAGATGGACCCG CGTCTGATTAGCTTGTTGGCGGGGTAACGGCCCACCAAGGCAACGATCAGTAGCCG ACCTGAGAGGGTGACCGGCCACATTGGGACTGAGACACGGCCCAG |
| 14 | rclust00715 | GATAAACGCTGGCGGCGTGCTTAACACATGCAAGTCGAACGAAGTTTTCTTTCGGG AGGAACTTAGTGGCGGACGGGTGAGTAACGCGTGGGTAACCTGCCCTGTACAGGGG GACAACAGCTGGAAACGGCTGCTAATACCGCATAAGCCCTTAGCACTGCATGGTGCA TAGGGAAAAGGAGCAATCCGGTACAGGATGGACCCGCGTCTGATTAGCCAGTTGGC AGGGTAACGGCCTACCAAAGCGACGATCAGTAGCCGATCTGAGAGGATGTACGGCC ACATTGGGACTGAGACACGGCCCAG |
| 15 | rclust00226 | ATTGAACGCTGGCGGCATGCTTTACACATGCAAGTCGAACGGCAGCACAGGGAGCT TGCTCCCGGGTGGCGAGTGGCGCACGGGTGAGTAATACATCGGAACGTGTCCTGTT GTGGGGGATAACTGCTCGAAAGGGTGGCTAATACCGCATGAGACCTGAGGGTGAAA GCGGGGGATCGCAAGACCTCGCGCAATTGGAGCGGCCGATGCCCGATTAGCTAGTT GGTGAGGTAAAGGCTCACCAAGGCGACGATCGGTAGCTGGTCTGAGAGGACGACC AGCCACACTGGGACTGAGACACGGCCCAG |
| 16 | unmap_OTU00273 | GATGAACGCTGGCGGCGTGCTTAACACATGCAAGTCGAACGAAGCACTTCGGACAG ATTCTTCGGATGAAGTCTTTGGTGACTGAGTGGCGGACGGGTGAGTAACGCGTGGG TAACCTGCCTCATACAGGGGGATAACAGTTAGAAATGGCTGCAAATACCGCATAAGC GCACGGTACTGCATGGTACAGTGTGAAAAACTCCGGTGGTATGAGATGGACCCGCG TTGGATTAGCTAGTTGGCAGGGTAACGGCCTACCAAGGCGACGATCCATAGCCGGCC TGAGAGGGTCGACGGCCACATTGGGACTGAGACACGGCCCAG |

**[Table 8]**

| SEQ ID NO: | OTU/cluster | Nucleotide sequence (5'→3') |
|---|---|---|
| 17 | rclust00268 | GATGAACGCTAGCTACAGGCTTAACACATGCAAGTCGAGGGGCAGCGGATTGAAG CTTGCTTCAATTGCCGGCGACCGGCGCACGGGTGAGTAACGCGTATCCAACCTTCCG CTTACTCGGGGATAGCCTTTCGAAAGAAAGATTAATACCCGATGGTATCTTAAGCAC GCATGAGATTAAGATTAAAGATTTATCGGTAAGCGATGGGGATGCGTTCCATTAGGCA GTTGGCGGGGTAACGGCCCACCAAACCTACGATGGATAGGGGTTCTGAGAGGAAGG TCCCCCACATTGGAACTGAGACACGGTCCAA |
| 18 | unmap_OTU00005 | GATGAACGCTGGCGGCGTGCTTAACACATGCAAGTCGAGCGAAGCAATCTAAGTGA AGTTTTCGGATGGATCTTAGATTGACTGAGCGGCGGACGGGTGAGTAACGCGTGGAT AACCTGCCTCACACAGGGGGATAACAGTTAGAAATGACTGCTAATACCGCATAAGCG CACAGTACCGCATGGTACAGTGTGAAAAACTCCGGTGGTGTGAGATGGATCCGCGT CTGATTAGGTAGTTGGTGGGGCAACGGCCCACCAAGCCGACGATCAGTAGCCGACC TGAGAGGGTGACCGGCCACATTGGGACTGAGACACGGCCCAA |
| 19 | rclust00467 | GATGAACGCTGGCGGCGTGCTTAACACATGCAAGTCGAACGGACGATGAAGAGCTT GCTCTTCAGAGTTAGTGGCGGACGGGTGAGTAACGCGTGGGTAACCTGCCTCATAC AGGGGGATAGCAGCTGGAAACGGCTGATAAAACCGCATAAGCGCACAGCATCGCAT GATGCAGTGTGAAAAACTCCGGTGGTATGAGATGGACCCGCGTCTGATTAGCTGGTT GGTGAGGTAACGGCCCACCAAGGCGACGATCAGTAGCCGGCCTGAGAGGGTGACC GGCCACATTGGGACTGAGACACGGCCCAA |
| 20 | unmap_OTU00644 | GATGAACGCTGGCGGCGTGCTTAACACATGCAAGTCGAGCGAAGCGATTTAAGTGA AGTTTTAGGATGGATCTTGGATTGACTGAGCGGCGGACGGGTGAGTAACGCGTGGAT AACCTGCCTCACACAGGGGGATAACAGTTAGAAATGACTGCTAATACCGCATAAGCG CACGGCATCGCATGATGCAGTGTGAAAAACTCCGGTGGTGTGAGATGGATCCGCG TCTGATTAGGTAGTTGGTGGGGTAACGGCCGACCAAGCCGACGATCAGTAGCCGAC CTGAGAGGGTGACCGGCCACATTGGGGACTGAGACACGGCCCAA |
| 21 | unmap_OTU00151 | GATGAACGCTGGCGGCGTGCCTAACACAAGCAAGTCGAGCGAAGCAATTTAAATGA GACTTCGGTGGATTTTAGATTGACTGAGCGGCGGACGGGTGAGTAACGCGTGGATA ACCTGCCTCACACAGGGGGATAACAGTTAGAAATGACTGCTAATACCGCATAAGCGC ACGGCATCGCATGATGCAGTGTGAAAACTCCGGTGGTGTGAGATGGATCCGCGTCT GATTAGGTAGTTGGTGAGGTAACGGCCCACCAAGCCGACGATCAGTAGCCGACCTG AGAGGGTGACCGGCCACATTGGGACTGAGACACGGCCCAA |
| 22 | rclust00255 | GATGAACGCTAGCTACAGGCTTAACACATGCAAGTCGAGGGGAAACGACATCGAAA GCTTGCTTTTGATGGGCGTCGACCGGCGCACGGGTGAGTAACGCGTATCCAACCTGC CCACCACTTGGGGATAACCTTGCGAAAGTAAGACTAATACCCAATGATATCTCTAGA AGACATCTGAAAGAGATTAAAGATTTATCGGTGATGGATGGGGATGCGGTCTGATTA GCTTGTTGGCGGGGTAACGGCCCACCAAGGCAACGATCAGTAGGGGTTCTGAGAGG AAGGTCCCCCACACTTGGAACTGAGACACGGTCCAA |
| 23 | rclust0012S | GACGAACGCTGGCGGCGTGCTTAACACATGCAAGTCGAACGGGGTGTTTATTTCGG TAAACACCAAGTGGCGAACGGGTGAGTAACGCGTAAGCAATCTACCTTCAAGATGG GGACAACACTTCGAAAGGGGTGCTAATACCGAATGAATGTAAGAGTATCGCATGAG ACACTTACTAAAGGAGGCCTCTGAAAATGCTTCCGCTTGAAGATGAGCTTGCGTCTG ATTAGCTAGTTGGTGAGGGTAAAGGCCCACCAAGGCGACGATCAGTAGCCGGTCTG AGAGGATGAACGGCCACATTGGGACTGAGACACGGCCCAGAC |

### [4. Long-term observation of intestinal bacterial flora]

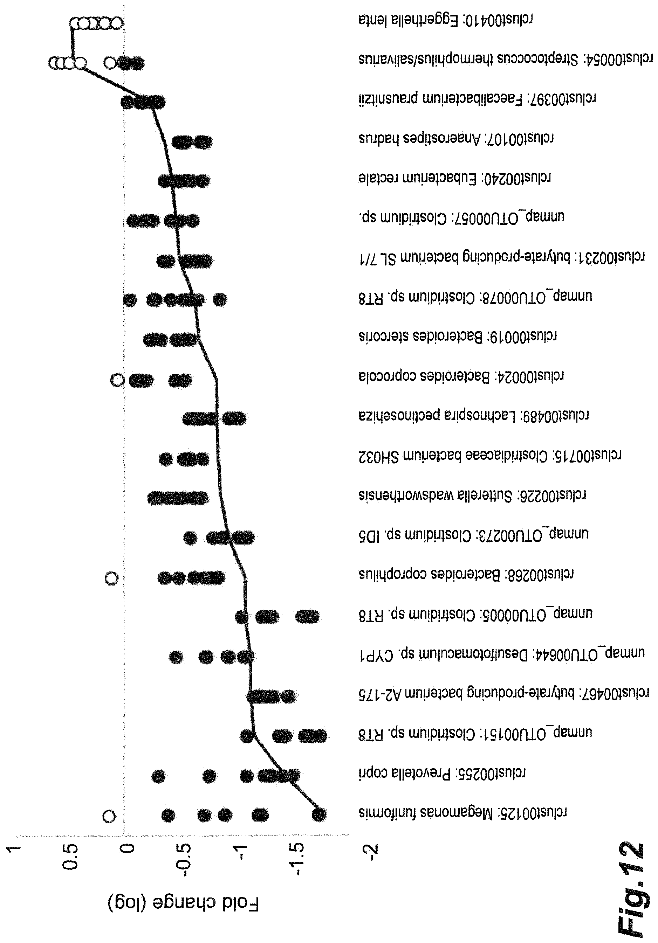

In order to evaluate the significant differences in the relative abundances of the identified twenty-one bacterial species, changes in the relative abundances of the various bacterial species were analyzed over a long time period, using fecal samples collected nine times, once in every two weeks, for the HC18 group (hereinafter, referred to as long-term HC18 group). Fig. 12 is a diagram showing the differences in the relative abundances of bacteria between the MS20 group and the long-term HC18 group (Log₁₀(average number of reads in MS20 group / average number of reads in long-term HC18 group)). The axis of ordinate in Fig. 12 represents the difference in the relative abundance of a bacterium.

In Fig. 12, an open circle (○) represents that the difference in the relative abundance is 0 or larger, and this means that the relative abundance is large in the MS20 group compared to the long-term HC18 group. On the other hand, a filled circle (●) represents that the difference in the relative abundance is less than 0, and this means that the relative abundance is small in the MS20 group compared to the long-term HC18 group. As is obvious from Fig. 12, the differences in the relative abundances of the identified twenty-one bacterial species showed a tendency that was similar to that in the case of making a comparison between MS20 group and HC40 group.

## Claims

1. A diagnosis method for an autoimmune disease, comprising:
a step of measuring relative abundances of bacteria included in a fecal sample collected from a test subject; and
a step of performing the following (1) or (2):
(1) in a case in which relative abundance of a bacterium whose nucleotide sequence of 16S ribosomal RNA gene has an identity of 99% or higher with nucleotide sequence set forth in SEQ ID NO:3 or SEQ ID NO:4, is large compared to the relative abundance in healthy subject, determining that the test subject has contracted, or has a high risk of contracting, the autoimmune disease; and
(2) in a case in which relative abundance of a bacterium whose nucleotide sequence of 16S ribosomal RNA gene has an identity of 99% or higher with any one of nucleotide sequences set forth in SEQ ID NO:5 to SEQ ID NO:23, is small compared to the relative abundance in healthy subject, determining that the test subject has contracted, or has a high risk of contracting, the autoimmune disease.

2. A diagnosis method for an autoimmune disease, comprising:
a step of measuring relative abundances of bacteria included in a fecal sample collected from a test subject before treatment and after treatment; and
a step of performing the following (3) or (4):
(3) in a case in which relative abundances before and after treatment of a bacterium whose nucleotide sequence of 16S ribosomal RNA gene has an identity of 99% or higher with nucleotide sequence set forth in SEQ ID NO:3 or SEQ ID NO:4, are compared, and the relative abundance after treatment is small compared to the relative abundance before treatment, determining that the disease state of the autoimmune disease of the test subject has been ameliorated by the treatment; and
(4) in a case in which relative abundances before and after treatment of a bacterium whose nucleotide sequence of 16S ribosomal RNA gene has an identity of 99% or higher with any one of nucleotide sequences set forth in SEQ ID NO:5 to SEQ ID NO:23 are compared, and the relative abundance after treatment is large compared to the relative abundance before treatment, determining that the disease state of the autoimmune disease of the test subject has been ameliorated by the treatment.

3. The diagnosis method according to claim 1 or 2, wherein the measurement of the relative abundances of bacteria includes comprehensive decoding of the nucleotide sequences of 16S ribosomal RNA gene of the bacteria included in the fecal sample.

4. The diagnosis method according to any one of claims 1 to 3, wherein the autoimmune disease is multiple sclerosis.

5. The diagnosis method according to claim 4, wherein the multiple sclerosis is relapsing-remitting multiple sclerosis.

6. A biomarker for autoimmune disease diagnosis, comprising an intestinal bacterium whose nucleotide sequence of 16S ribosomal RNA gene has an identity of 99% or higher with any one of nucleotide sequences set forth in SEQ ID NO:3 to SEQ ID NO:23.

7. Use of an intestinal bacterium whose nucleotide sequence of 16S ribosomal RNA gene has an identity of 99% or higher with any one of nucleotide sequences set forth in SEQ ID NO:3 to SEQ ID NO:23, as a biomarker for autoimmune disease diagnosis.

8. A preventing or treating agent for an autoimmune disease, comprising, as an active ingredient, at least one selected from the group consisting of a bacterium whose nucleotide sequence of 16S ribosomal RNA gene has an identity of 99% or higher with any one of nucleotide sequences set forth in SEQ ID NO:5 to SEQ ID NO:23, and a physiologically active substance derived from the bacterium.


SEQUENCE LISTING

<110>  National Center of Neurology and Psychiatry
       The University of Tokyo
       Azabu University
       Juntendo University

<120>  A method for the diagnosis of autoimmune disease, a biomarker for
        the diagnosis of autoimmune disease, and  an  agent for
       prevention or curing autoimmune disease

<130>  FP15-0494-00

<160>  23

<170>  PatentIn version 3.5

<210>  1
<211>  20
<212>  DNA
<213>  Artificial Sequence

<220>
<223>  Forward primer 27Fmod

<400>  1
agrgtttgat ymtggctcag                                                   20


<210>  2
<211>  19
<212>  DNA
<213>  Artificial Sequence

<220>
<223>  Reverse primer 338R

<400>  2
tgctgcctcc cgtaggagt                                                    19


<210>  3
<211>  307
<212>  DNA
<213>  Eggerthella lenta

<400>  3
gatgaacgct ggcggcgtgc ctaacacatg caagtcgaac gatgaaaccg ccctcgggcg      60

gacatgaagt ggcgaacggg tgagtaacac gtgaccaacc tgcccccctc tccgggacaa      120

ccttgggaaa ccgaggctaa taccggatac tccctcccct gctcctgcag gggtcgggaa     180

agcccaggcg gagggggatg gggtcgcggc ccattaggta gtaggcgggg taacggccca      240

cctagcccgc gatgggtagc cgggttgaga gaccgaccgg ccacattggg actgagatac     300

ggcccag                                                                307


<210>  4
<211>  316
<212>  DNA

<213>  Streptococcus thermophilus

<400>  4
gacgaacgct ggcggcgtgc ctaatacatg caagtagaac gctgaagaga ggagcttgct      60

cttcttggat gagttgcgaa cgggtgagta acgcgtaggt aacctgcctt gtagcggggg     120

ataactattg gaaacgatag ctaataccgc ataacaatgg atgacacatg tcatttattt     180

gaaaggggca attgctccac tacaagatgg acctgcgttg tattagctag taggtgaggt     240

aatggctcac ctaggcgacg atacatagcc gacctgagag ggtgatcggc cacactggga     300

ctgagacacg gcccag                                                     316


<210>  5
<211>  309
<212>  DNA
<213>  Faecalibacterium prausnitzii

<400>  5
gacgaacgct ggcggcgcgc ctaacacatg caagtcgaac gagagatgag gagcttgctc      60

ttcaaatcga gtggcgaacg ggtgagtaac gcgtgaggaa cctgcctcaa agagggggac     120

aacagttgga aacgactgct aataccgcat aagcccacgg ctcggcatcg agcagaggaa     180

aggagtgatc cgctttgaga tggcctcgcg tccgattagc tagttggtga ggtaacggcc     240

caccaaggcg acgatcggta gccggactga gaggttgaac ggccacattg ggactgagac     300

acggcccag                                                             309


<210>  6
<211>  326
<212>  DNA
<213>  Anaerostipes hadrus

<400>  6
gatgaacgct ggcggcgtgc ttaacacatg caagtcgaac gaaacacctt atttgatttt      60

cttcggaact gaagatttgg tgattgagtg gcggacgggt gagtaacgcg tgggtaacct     120

gccctgtaca gggggataac agtcagaaat gactgctaat accgcataag accacagcac     180

cgcatggtgc aggggtaaaa actccggtgg tacaggatgg acccgcgtct gattagctgg     240

ttggtgaggt aacggctcac caaggcgacg atcagtagcc ggcttgagag agtgaacggc     300

cacattggga ctgagacacg gcccaa                                          326


<210>  7
<211>  326
<212>  DNA
<213>  Eubacterium rectale

<400>  7
gatgaacgct ggcggcgtgc ttaacacatg caagtcgaac gaagcacttt atttgatttc      60

cttcgggact gattattttg tgactgagtg gcggacgggt gagtaacgcg tgggtaacct     120

gccttgtaca gggggataac agttggaaac ggctgctaat accgcataag cgcacggcat 180

cgcatgatgc agtgtgaaaa actccggtgg tataagatgg acccgcgttg gattagctag 240

ttggtgaggt aacggcccac caaggcgacg atccatagcc gacctgagag ggtgaccggc 300

cacattggga ctgagacacg gcccaa 326

<210> 8
<211> 324
<212> DNA
<213> Clostridium sp.

<220>
<221> misc_feature
<222> (284)..(284)
<223> n is a, c, g, or t

<400> 8
gatgaacgct ggcggcgtgc ttaacacatg caagtcgaac gaagcaatct aacggaagtt 60

ttcggatgga agctggattg actgagtggc ggacgggtga gtaacgcgtg ggtaacctgc 120

ctcacactgg gggacaacag ttagaaatga ctgctaatac cgcataagcg cacaggaccg 180

catggtccgg tgtgaaaaac tctagtggtg tgagatggac ccgcgtttga ttagctagtt 240

ggtggggtaa cggcctacca aggcgacgat caatagccga cctnagaggg tgaccggcca 300

cattgggact gagacacggc ccaa 324

<210> 9
<211> 323
<212> DNA
<213> Coprococcus sp.

<400> 9
gatgaacgct ggcggcgtgc ttaacacatg caagtcgaac gaagcactta tctttgaatc 60

ttcggatgaa gaggtttgtg actgagtggc ggacgggtga gtaacgcgtg ggtaacctgc 120

ctcatacagg gggataacag ttagaaatga ctgctaatac cgcataagac cacggagccg 180

catggctcag tgggaaaaac tccggtggta tgagatggac ccgcgtctga ttaggtagtt 240

ggtggggtaa cggcctacca agccaacgat cagtagccga cctgagaggt gaccggccac 300

attgggactg agacacggcc caa 323

<210> 10
<211> 319
<212> DNA
<213> Clostridium sp.

<400> 10
gatgaacgct ggcggcgtgc ttaacacatg caagtcgagc gaagcgattt aaatgagact 60

tcggtggatt ttaaattgac tgagcggcgg acgggtgagt aacgcgtgga taacctgcct 120

cacacagggg gataacagtt agaaatgact gctaataccg cataagcgca cggtaccgca 180

tggtacagtg cgaaaaactc cggtggtgtg agatggatcc gcgtctgatt aggtagttgg 240

tgaggtaacg gcccacaagc cgacgatcag tagccgacct gagaggtgac cggcacattg 300

ggactgagac acggcccag 319

<210> 11
<211> 314
<212> DNA
<213> Bacteroides stercoris

<400> 11
gatgaacgct agctacaggc ttaacacatg caagtcgagg ggcagcatca tcaaagcttg 60

ctttgatgga tggcgaccgg cgcacgggtg agtaacacgt atccaacctg ccgacaacac 120

tgggatagcc tttcgaaaga aagattaata ccggatggca tagttttccc gcatgggata 180

attattaaag aatttcggtt gtcgatgggg atgcgttcca ttaggcagtt ggcggggtaa 240

cggcccacca aaccaacgat ggataggggt tctgagagga aggtccccca cattggaact 300

gagacacggt ccaa 314

<210> 12
<211> 315
<212> DNA
<213> Bacteroides coprocola

<400> 12
gatgaacgct agctacaggc ttaacacatg caagtcgagg ggcagcatga acttagcttg 60

ctaagtttga tggcgaccgg cgcacgggtg agtaacacgt atccaacctt ccgtttactc 120

agggatagcc tttcgaaaga aagattaata cctgatagta tggtgagatt gcatgatagc 180

accattaaag atttattggt aaacgatggg gatgcgttcc attaggtagt aggcggggta 240

acggcccacc tagccgacga tggatagggg ttctgagagg aaggtccccc acattggaac 300

tgagacacgg tccaa 315

<210> 13
<211> 326
<212> DNA
<213> Lachnospira sp.

<400> 13
gatgaacgct ggcggcgtgc ttaacacatg caagtcgaac gaagcattta agacggattc 60

tttcgggatg aagactttta tgactgagtg gcggacgggt gagtaacgcg tgggtaacct 120

gcctcacaca gggggatagc agttggaaac ggctgataat accgcataag cgcacagtac 180

cgcatggtac agtgtgaaaa actccggtgg tgtgagatgg acccgcgtct gattagcttg 240

ttggcggggt aacggcccac caaggcaacg atcagtagcc gacctgagag ggtgaccggc 300

cacattggga ctgagacacg gcccag                                          326

<210>  14
<211>  307
<212>  DNA
<213>  Unknown

<220>
<223>  Clostridium cluster XIVa

<400>  14
gataaacgct ggcggcgtgc ttaacacatg caagtcgaac gaagtttttc tttcgggagg      60

aacttagtgg cggacgggtg agtaacgcgt gggtaacctg ccctgtacag ggggacaaca     120

gctggaaacg gctgctaata ccgcataagc ccttagcact gcatggtgca tagggaaaag     180

gagcaatccg gtacaggatg gacccgcgtc tgattagcca gttggcaggg taacggccta     240

ccaaagcgac gatcagtagc cgatctgaga ggatgtacgg ccacattggg actgagacac     300

ggcccag                                                               307

<210>  15
<211>  308
<212>  DNA
<213>  Sutterella wadsworthensis

<400>  15
attgaacgct ggcggcatgc tttacacatg caagtcgaac ggcagcacag ggagcttgct      60

cccgggtggc gagtggcgca cgggtgagta atacatcgga acgtgtcctg ttgtggggga     120

taactgctcg aaagggtggc taataccgca tgagacctga gggtgaaagc gggggatcgc     180

aagacctcgc gcaattggag cggccgatgc ccgattagct agttggtgag gtaaaggctc     240

accaaggcga cgatcggtag ctggtctgag aggacgacca gccacactgg gactgagaca     300

cggcccag                                                              308

<210>  16
<211>  324
<212>  DNA
<213>  Clostridium sp.

<400>  16
gatgaacgct ggcggcgtgc ttaacacatg caagtcgaac gaagcacttc ggacagattc      60

ttcggatgaa gtctttggtg actgagtggc ggacgggtga gtaacgcgtg ggtaacctgc     120

ctcatacagg gggataacag ttagaaatgg ctgcaaatac cgcataagcg cacggtactg     180

catggtacag tgtgaaaaac tccggtggta tgagatggac ccgcgttgga ttagctagtt     240

ggcagggtaa cggcctacca aggcgacgat ccatagccgg cctgagaggg tcgacggcca     300

cattgggact gagacacggc ccag                                            324

<210>  17
<211>  315
<212>  DNA
<213>  Bacteroides coprophilus

<400>  17
gatgaacgct agctacaggc ttaacacatg caagtcgagg ggcagcggga ttgaagcttg      60

cttcaattgc cggcgaccgg cgcacgggtg agtaacgcgt atccaacctt ccgcttactc     120

ggggatagcc tttcgaaaga aagattaata cccgatggta tcttaagcac gcatgagatt     180

aagattaaag atttatcggt aagcgatggg gatgcgttcc attaggcagt tggcggggta     240

acggcccacc aaacctacga tggatagggg ttctgagagg aaggtccccc acattggaac     300

tgagacacgg tccaa                                                      315


<210>  18
<211>  323
<212>  DNA
<213>  Clostridium sp.

<400>  18
gatgaacgct ggcggcgtgc ttaacacatg caagtcgagc gaagcaatct aagtgaagtt      60

ttcggatgga tcttagattg actgagcggc ggacgggtga gtaacgcgtg gataacctgc     120

ctcacacagg gggataacag ttagaaatga ctgctaatac cgcataagcg cacagtaccg     180

catggtacag tgtgaaaaac tccggtggtg tgagatggat ccgcgtctga ttaggtagtt     240

ggtggggcaa cggcccacca agccgacgat cagtagccga cctgagaggt gaccggccac     300

attgggactg agacacggcc caa                                             323


<210>  19
<211>  309
<212>  DNA
<213>  Coprococcus sp.

<400>  19
gatgaacgct ggcggcgtgc ttaacacatg caagtcgaac ggacgatgaa gagcttgctc      60

ttcagagtta gtggcggacg ggtgagtaac gcgtgggtaa cctgcctcat acagggggat     120

agcagctgga aacggctgat aaaaccgcat aagcgcacag catcgcatga tgcagtgtga     180

aaaactccgg tggtatgaga tggacccgcg tctgattagc tggttggtga ggtaacggcc     240

caccaaggcg acgatcagta gccggcctga gagggtgacc ggccacattg ggactgagac     300

acggcccaa                                                             309


<210>  20
<211>  326
<212>  DNA
<213>  Desulfotomaculum sp.

<400> 20
gatgaacgct ggcggcgtgc ttaacacatg caagtcgagc gaagcgattt aagtgaagtt 60

ttaggatgga tcttggattg actgagcggc ggacgggtga gtaacgcgtg gataacctgc 120

ctcacacagg gggataacag ttagaaatga ctgctaatac cgcataagcg cacggcatcg 180

catgatgcag tgtgaaaaaa ctccggtggt gtgagatgga tccgcgtctg attaggtagt 240

tggtggggta acggccgacc aagccgacga tcagtagccg acctgagagg gtgaccggcc 300

acattgggga ctgagacacg gcccaa 326

<210> 21
<211> 321
<212> DNA
<213> Clostridium sp.

<400> 21
gatgaacgct ggcggcgtgc ctaacacaag caagtcgagc gaagcaattt aaatgagact 60

tcggtggatt ttagattgac tgagcggcgg acgggtgagt aacgcgtgga taacctgcct 120

cacacagggg gataacagtt agaaatgact gctaataccg cataagcgca cggcatcgca 180

tgatgcagtg tgaaaactcc ggtggtgtga gatggatccg cgtctgatta ggtagttggt 240

gaggtaacgg cccaccaagc cgacgatcag tagccgacct gagagggtga ccggccacat 300

tgggactgag acacggccca a 321

<210> 22
<211> 319
<212> DNA
<213> Prevotella copri

<400> 22
gatgaacgct agctacaggc ttaacacatg caagtcgagg ggaaacgaca tcgaaagctt 60

gcttttgatg ggcgtcgacc ggcgcacggg tgagtaacgc gtatccaacc tgcccaccac 120

ttggggataa ccttgcgaaa gtaagactaa tacccaatga tatctctaga agacatctga 180

aagagattaa agatttatcg gtgatggatg gggatgcggt ctgattagct tgttggcggg 240

gtaacggccc accaaggcaa cgatcagtag gggttctgag aggaaggtcc cccacacttg 300

gaactgagac acggtccaa 319

<210> 23
<211> 323
<212> DNA
<213> Megamonas funiformis

<400> 23
gacgaacgct ggcggcgtgc ttaacacatg caagtcgaac ggggtgttta tttcggtaaa 60

caccaagtgg cgaacgggtg agtaacgcgt aagcaatcta ccttcaagat ggggacaaca 120

cttcgaaagg ggtgctaata ccgaatgaat gtaagagtat cgcatgagac acttactaaa 180

ggaggcctct gaaaatgctt ccgcttgaag atgagcttgc gtctgattag ctagttggtg 240

agggtaaagg cccaccaagg cgacgatcag tagccggtct gagaggatga acggccacat 300

tgggactgag acacggccca gac 323